# EUROPEAN PATENT APPLICATION

(11) **EP 4 273 242 A1**
(43) Date of publication of application: **08.11.2023**
(21) Application number: 22171430.6
(22) Date of filing: 03.05.2022
(51) Int. Cl.: C12N 15/11

(54) **CRISPR-BASED MODIFICATION OF HUMAN HBD GENE**

(71) Applicant: ETH Zurich, 8092 Zurich (CH)
(72) Inventor: Corn, Jacob, 8057 Zurich (CH); Boontanrart, Mandy, 8057 Zurich (CH)

(57) **Abstract**

The present invention relates to the field of CRISPR-based genome editing. The invention provides nucleic acids, compositions and kits for editing a human HBD gene and their use in the treatment of haemoglobinopathies. The invention further provides methods of making thereof and methods of editing a human *HBD* gene.

## Description

### Field of the invention

The present invention relates to the field of CRISPR-based genome editing. The invention provides nucleic acids, compositions and kits for editing a human *HBD* gene and their use in the treatment of haemoglobinopathies. The invention further provides methods of making thereof and methods of editing a human *HBD* gene.

### Background of the invention

Red blood cells (RBCs) are involved in several blood diseases, collectively termed haemoglobinopathies. Haemoglobinopathies are among the most common inherited diseases in the world [E. Kohne MEDICINE, Deutsches Ärzteblatt International 108(31-32): 532-40 (2011)]. The World Health Organization (WHO) reports that about 7% of the world's population carries a gene mutation related to haemoglobinopathy. It is estimated that around 350,000 affected children are born each year alone [Williams, T. N. & Weatherall, D. J. World Distribution, Population Genetics, and Health Burden of the Hemoglobinopathies. Cold Spring Harb Perspect Med 2, (2012)]. Some of these diseases are characterized by a low oxygen-carrying capacity of the blood due to a low number or an abnormality of RBCs or haemoglobin. These are collectively referred to as anemias, such as beta-thalassemia and sickle cell disease (SCD) [Thachil, J., Owusu-Ofori, S. & Bates, I. Haematological Diseases in the Tropics. Manson's Tropical Infectious Diseases 894-932.e7 (2014) doi:10.1016/B978-0-7020-5101-2.00066-2].

Beta-thalassemic genetic disorders are generally caused by a disruption of beta-globin expression, resulting in loss of Haemoglobin A (HbA) and a dramatic reduction in life expectancy [Galanello, R. et al. Erythropoiesis following bone marrow transplantation from donors heterozygous for β-thalassaemia. British Journal of Haematology (1989) doi:10.1111/j.1365-2141.1989.tb04324.x].

In SCD, haemoglobin becomes irregular due to a single mutation in the beta-globin chain, which, in turn, leads to misshaped (sickle-shaped) RBCs. Said irregular haemoglobin is known as sickle cell haemoglobin (HbS). These sickle shaped RBCs are more rigid and less viscoelastic than regular RBCs, which can lead to blood vessel blockage, pain, strokes, and other tissue damage [Rees, D. C., Williams, T. N. & Gladwin, M. T. Sickle-cell disease. Lancet 376, 2018-2031 (2010)].

Efforts to develop novel treatment options for haemoglobinopathies have largely focussed on methods of increasing levels of fetal haemoglobin (HbF) in order to compensate for reduced levels of regular HbA.

For example, re-expression of the gamma-globin subunit of HbF (encoded by the *HBG* gene), which is typically silenced after birth, has been described for the treatment of beta-thalassemia. Upon re-expression, gamma-globin associates with alpha-globin (encoded by the *HBA* gene), thereby forming the HbF complex consisting of two alpha-globin and two gamma-globin subunits. It has been further shown that reactivation of HbF may attenuate effects associated with haemoglobinopathies [Wienert, B., Martyn, G. E., Funnell, A. P. W., Quinlan, K. G. R. & Crossley, M. Wake-up Sleepy Gene: Reactivating Fetal Globin for β-Hemogiobinopathies. Trends in Genetics (2018) doi:10.1016/j.tig.2018.09.004].

Re-expression of HbF has been achieved early on by bone marrow transplantation [Alter, B. P. Fetal erythropoiesis in stress hematopoiesis. Experimental Hematology (1979)] as well as by treatment using small molecules, such as hydroxyurea [Platt, O. S., Orkin, S. H. & Dover, G. Hydroxyurea enhanced fetal hemoglobin production in sickle cell anemia. Journal of Clinical Investigation (1984) doi:10.1172/JCl111464]. More recently genome editing technologies, particularly CRISPR-Cas9 mediated genome editing, have been used to increase HbF levels by downregulating expression levels of the transcription factor BCL11A - which contributes to suppression of HbF - by targeting erythroid-specific elements in the BCL11A enhancer region [Frangoul, H. et al. CRISPR-Cas9 Gene Editing for Sickle Cell Disease and β-Thaiassemia. N Engl J Med 384, 252-260 (2021)]. Alternatively, HbF levels have been increased by modifying ZBTB7A or BCL11A binding sites within the *HBG* gene promoter region [Martyn, G., Wienert B. et al., Nat. Genet. 2018; 50(4):498-503]. The transcription factors BCL11A and ZBTB7A generally repress expression of HBG genes by binding directly to its proximal promoter region, which is the region approximately 115 to 200 basepairs (bp) upstream of the transcription start site. HBG expression is controlled by the activator Kruppel-like transcription factor 1 (KLF1), which additionally promotes transcription of the *HBB* gene. It is thus generally known that single point mutations, such as those found in SCD, can be repaired by CRISPR-Cas9 associated homology-directed repair (HDR; also referred to as homology-guided repair) [Yeh, C. D., Richardson, C. D. & Corn, J. E. Advances in genome editing through control of DNA repair pathways. Nature Cell Biology (2019) doi:10.1038/s41556-019-0425-z].

However, while most studies have focussed on increasing levels of fetal HbF, relatively little research has been performed on increasing expression of haemoglobin A2 (HbA2) to compensate for low/abnormal HbA expression. For example, HbA2 has been shown to inhibit polymerization of haemoglobin S (HbS) by acting as an anti-sickling agent [Poillon, W. N., Kim, B. C., Rodgers, G. P., Noguchi, C. T. & Schechter, A. N. Sparing effect of hemoglobin F and hemoglobin A2 on the polymerization of hemoglobin S at physiologic ligand saturations. Proc Natl Acad Sci U S A 90, 5039-5043 (1993)].

HbA is a tetrameric protein comprised of 2 alpha-globin subunits and 2 beta-globin subunits and accounts for approximately 97% of total haemoglobin in adults.

HbA2 is a tetrameric protein comprised of 2 alpha-globin subunits and 2 delta-globin subunits and accounts for approximately 3% of total haemoglobin in adults. It has been shown that the discrepancy in expression levels between HbA and HbA2 is not due to differences in protein stability of delta- and beta-globin or differences in translation. Instead delta-globin is synthesized with lower transcription [Steinberg, M. H. and G. P. Rodgers (2015), Br J Haematol 170(6): 781-787].

The delta-globin subunit present in HbA2 is encoded by the *HBD* gene, which is located on the same chromosome as *HBB* and its expression is regulated by the same control region [Somervaille, T. Disorders of Hemoglobin: Genetics, Pathophysiology, and Clinical Management. J R Soc Med 94, 602-603 (2001)].

Genetic studies have suggested that low expression of delta-globin protein in adult blood is due to mutations in the KLF1 binding site (CACCC box) within the proximal *HBD* promoter region. It is known that Kruppel-like factors, such as KLF1, play an important role in maturation of RBCs. Further transgenic studies have shown that human *HBD* gene can be activated *in vivo* by insertion of a KLF1 binding site into the *HBD* promoter region [Ristaldi, M. S. et al. Activation of the delta-globin gene by the beta-globin gene CACCC motif. Blood Cells Mol Dis 25, 193-209 (1999)].

However, previous studies on increasing *HBD* expression have typically been transgenic experiments and have not been performed in a context in which both *HBB* and *HBD* genes are present, as is the case at the endogenous globin locus.

There is still an unmet medical need to provide improved therapies for the treatment of haemoglobinopathies, e.g. anemias, particularly SCD and beta-thalassemia.

### Description of the invention

Thus, it is an **object of the present invention** to address this need. The objective is achieved by a CRISPR/Cas composition or a kit for editing a human *HBD* gene as defined in claim 1. Further aspects of the invention are disclosed in the specification and independent claims, preferred embodiments are disclosed in the specification and the dependent claims.

The present invention will be described in more detail below. Briefly, the invention provides:
- In a first aspect, a CRISPR/Cas composition (comprising components (a), (b) and (c)) suitable for editing a human HBD gene;
- In a second aspect, a CRISPR/Cas kit (comprising components (a), (b) and (c)) suitable for editing a human HBD gene;
- In a third aspect, a gRNA (component a) that is suitable for editing a human *HBD* gene;
- In a fourth aspect, a DNA donor template and a vector comprising such DNA donor template (component b) that are suitable for editing a human HBD gene;
- In a fifth aspect, specific nucleic acids, such as vectors (comprising component (a) and/or (b), and optionally (c));
- In a sixth aspect, a method manufacturing a gRNA (component a);
- In a seventh aspect, a method of editing a human *HBD* gene in a cell, *in vitro* or ex *vivo;*
- In an eighth aspect, the use of the CRISPR/Cas composition, the kit and the individual components described herein in medicine.

It is understood that the various embodiments/features, preferences and ranges as provided/disclosed in this specification may be combined at will as long as the specific combination of the embodiments/features is technically meaningful. It is further understood, that the definitions and explanations provided in the first aspect are likewise applicable to the remaining aspects of the invention (second to eights aspect). Further, depending on the specific embodiment, selected **definitions**, embodiments or ranges may not apply.

Unless otherwise stated, the following **definitions** shall apply in this specification:
As used herein, the term "a", "an", "the" and similar terms used in the context of the present invention (especially in the context of the claims) are to be construed to cover both the singular and plural unless otherwise indicated herein or clearly contradicted by the context.

As used herein, the term "and/or" means that either all or only one of the elements of said group may be present. For example, "A and/or B" means "only A, or only B, or both A and B". In the case of "only A", the term also covers the possibility that B is absent, i.e. "only A, but not B".

As used herein, the terms "including", "containing" and "comprising" are used herein in their open-ended, non-limiting sense. It is understood that the various embodiments, preferences and ranges may be combined at will.

Operably linked: As used herein, the term "operably linked" means that the nucleotide sequence of interest is linked to regulatory sequence(s), e.g. a promoter sequence, in a manner which allows for expression of the nucleotide sequence. The skilled person understands that selecting a suitable promoter sequence depends on several factors including the target cell in which the nucleotide sequence should be expressed, the length of the nucleotide sequence to be expressed and the desired expression level. Suitable promoter sequences that are functional in a mammalian cell are for example promoter sequences from cytomegalovirus (CMV) immediate-early, herpes simplex virus (HSV) thymidine kinase, simian virus (SV40) promoter, long terminal repeats (LTRs) retroviral promoter, human elongation factor-1 promoter (EF1), Rous sarcoma virus (RSV) promoter, mouse mammarytumorvirus (MMTV) promoter murine stem cell virus promoter (MSCV), phosphoglycerate kinase-1 locus promoter (PGK) and mouse metallothionein-I.

Particularly suitable promoters for the expression for small RNAs such as guide RNAs according to the present invention are for example U6 promoter and H1 promoter. The skilled person is able to select a suitable promoter sequence based on the cell type and desired expression level.

Treatment: As used herein, the terms "treating", "treat" and "treatment" include one or more of the following: (i) preventing a disease, pathologic or medical condition from occurring (e.g. prophylaxis); (ii) inhibiting the disease, pathologic or medical condition or arresting its development; (iii) relieving the disease, pathologic or medical condition; (iv) diminishing symptoms associated with the disease, pathologic or medical condition. Thus, the terms "treat", "treatment", and "treating" extend to prophylaxis and include prevent, prevention, preventing, lowering, stopping or reversing the progression or severity of the condition or symptoms being treated. As such, the term "treatment" includes medical, therapeutic, and/or prophylactic administration, as appropriate.

Gene: As used herein a "gene", refers to a DNA region (including exons and introns) encoding a gene product, as well as all DNA regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites, and locus control regions.

Nucleic acid / polynucleotide: The terms "nucleic acid" and "polynucleotide" refer to a deoxyribonucleotide or ribonucleotide polymer, in linear or circular conformation, and in either single- or double-stranded form. As used herein, these terms are not to be construed as limiting with respect to the length of a polymer. The terms can encompass known analogs of natural nucleotides, as well as nucleotides that are modified in the base, sugar and/or phosphate moieties (e.g. phosphorothioate or phosphorodithioate backbones).

Nucleotide: The term "nucleotide" refers to deoxyribonucleotides or ribonucleotides. The nucleotides may be standard nucleotides (i.e., adenosine, guanosine, cytidine, thymidine, and uridine) or nucleotide analogs. A nucleotide analog refers to a nucleotide having a modified purine or pyrimidine base or a modified ribose moiety. A nucleotide analog may be a naturally occurring nucleotide (e.g., inosine) or a non-naturally occurring nucleotide. Examples of non-naturally occurring nucleotides include nucleotides that are modified at the 2'-O position of the sugar such as 2'-OMe, 2'-MOE, 2'-F modified nucleotides, as well as locked nucleic acids (LNAs), peptide nucleic acids (PNA), and morpholinos. The skilled person understands that this list of modified nucleotides is not exhaustive and is able to select suitable modified nucleotides based on the present disclosure.

Polypeptide: The terms "polypeptide" and "protein" are used interchangeably and refer to a polymer of amino acid residues.

Throughout this specification a number of abbreviations are used, including:
- 2'-F: 2'-Fluoro
- 2'-MOE: 2'-methoxyethyl
- 2'-OMe: 2'-O-methyl
- AAV: Adeno-associated virus
- b-DRF: beta-globin Direct Repeat Factor (also referred to as b-DRE: beta-globin Direct Repeat Element)
- CMV: Cytomegalovirus
- EF-1: Human elongation factor 1
- HBA: Gene encoding alpha-globin
- HbA: Haemoglobin A (synonymously haemoglobin A1)
- HbA2: Haemoglobin A2
- HBB: Gene encoding beta-globin
- HBD: Gene encoding delta-globin
- HbF: Fetal haemoglobin
- HBG: Gene encoding gamma-globin
- HSPCs: Human hematopoietic stem and progenitor cells
- HSV: Herpes simplex virus
- IVT: *in vitro* transcription
- KLF1: Kruppel-Life Factor 1
- LNA: Locked nucleic acid
- LTRs: long terminal repeats
- mAU: Milli absorbance unit (arbitrary unit)
- mPB-HSPCs: Mobilized peripheral blood hematopoietic stem and progenitor cells
- MMTV: Mouse mammarytumorvirus
- MSCV: Murine stem cell virus
- PGK: Phosphoglycerate kinase-1
- PNA: Peptide nucleic acid
- RNP: Ribonucleoprotein complex
- RSV: Rous sarcoma virus
- SCD: Sickle cell disease
- ssODN: single strand oligodeoxynucleotide
- TFIIB: Transcription Factor II B
- WT: Wild type

### Description of the figures

**Figure 1****: Comparison of the *HBB* (top) and *HBD* (bottom) gene promoter sequences.**
   Underlined sequences refer to promoter elements in the *HBB* promoter sequence. Underlined are KLF1, CP1, b-DRF, TFIIB, and TATA-box sites. Of note, the *HBD* promoter only has an intact TATA-box sequence, while all other underlined sites are incomplete.
**Figure 2****. qRT-PCR data (*HBD* expression) after CRISPR/Cas9 RNP editing with DNA donor templates**
   HUDEP2 cells were edited with ribonucleoprotein complexes (RNP) of SpCas9 (SEQ ID NO: 56) and gRNA in the presence of different DNA donor templates. The gRNA used in this experiment had SEQ ID NO: 49, which comprises the DNA targeting segment having SEQ ID NO: 26.
   Conditions were as follows:
      1. Cas9 + gRNA, no DNA donor templates
      2. Cas9 + gRNA + DNA donor template containing only KLF1 binding site (KLF1 binding site having SEQ ID NO: 52; DNA donor template having SEQ ID NO: 64)
      3. Cas9 + gRNA+ DNA donor template containing only the b-DRF motif and the TFIIB binding site (b-DRF motif having SEQ ID NO: 54 and TFIIB binding site having SEQ ID NO: 53; DNA donor template having SEQ ID NO: 65)
      4. Cas9 + gRNA+ DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55).
   Edited cells were nucleofected and differentiated for 5 days and then harvested for analysis. The data is presented as mean ± SD of three biological replicates. p value indicates a paired, two-tailed Student's t test (ns, nonsignificant; *p % 0.05; **p % 0.01; ***p % 0.001).
   Y-axis shows % of HBD of beta-like globins. The beta-like globins measured include HBB, HBD, and HBG. HBD is shown as a percentage of the total of these globin genes.

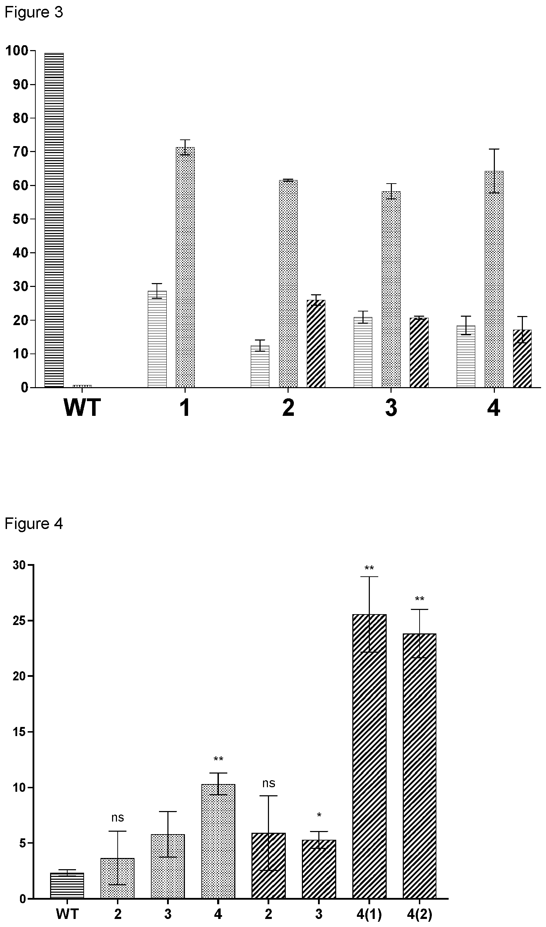

**Figure 3****. Editing Outcomes of Editing HUDEP2 cells**
   Editing Efficiencies in HUDEP2 cells (measured by NGS)
   HUDEP2 cells were edited with Cas9 + gRNA RNP complexes with different DNA donor templates. Conditions were as follows:
      1. Cas9 + gRNA, no DNA donor templates
      2. Cas9 + gRNA + DNA donor template containing only KLF1 binding site (KLF1 binding site having SEQ ID NO: 52; DNA donor template having SEQ ID NO: 64)
      3. Cas9 + gRNA+ DNA donor template containing only the b-DRF motif and the TFIIB binding site (b-DRF motif having SEQ ID NO: 54 and TFIIB binding site having SEQ ID NO: 53; DNA donor template having SEQ ID NO: 65)
      4. Cas9 + gRNA+ DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55).
   Cells were harvested for NGS sequencing and CRISPResso was used to assign editing outcomes of percent unmodified, NHEJ, and HDR. The data is presented as mean ± SD of three biological replicates. p value indicates a paired, two-tailed Student's t test (ns, nonsignificant; *p % 0.05; **p % 0.01; ***p % 0.001).
   Y-axis shows the % of editing outcomes as either unmodified, NHEJ, or HDR. Horizontal line bars indicate unmodified alleles, dot bars indicate NHEJ alleles, and slanted line bars indicate HDR alleles.
**Figure 4****. qRT-PCR data (*HBD* expression) of heterozygous and homozygous knockin clones.**
   Heterozygous and homozygous clones were isolated from editing conditions 2 (Cas9 + gRNA + DNA donor template containing only KLF1 binding site (KLF1 binding site having SEQ ID NO: 52; DNA donor template having SEQ ID NO: 64)), 3 (Cas9 + gRNA+ DNA donor template containing only the b-DRF motif and the TFIIB binding site (b-DRF motif having SEQ ID NO: 54 and TFIIB binding site having SEQ ID NO: 53; DNA donor template having SEQ ID NO: 65)) and 4 (Cas9 + gRNA+ DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55)) shown in Fig. 4. These clones were differentiated and harvested for qRT-PCR. For condition 4, two homozygous clones were isolated, denoted by 4(1) and 4(2). The wildtype condition is depicted in the bar with horizontal black lines. Heterozygous clones are depicted in bars with black dots. Homozygous clones are depicted in bars with slanted black lines. The data is presented as mean ± SD of three biological replicates. p value indicates a paired, two-tailed Student's t test (ns, nonsignificant; *p % 0.05; **p % 0.01; ***p % 0.001).
   Y-axis shows % of HBD of beta-like globins. The beta-like globins measured include HBB, HBD, and HBG. HBD is shown as a percentage of the total of these globin genes.

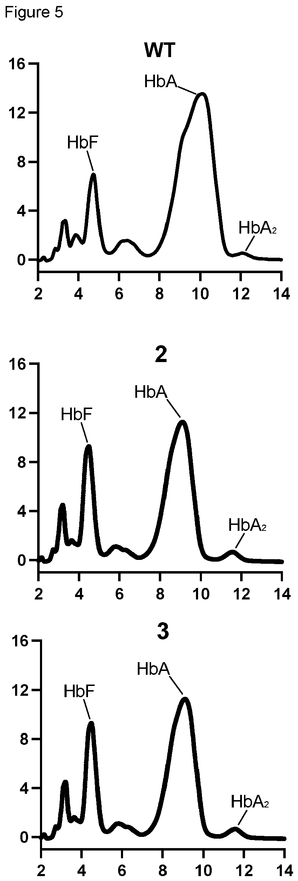

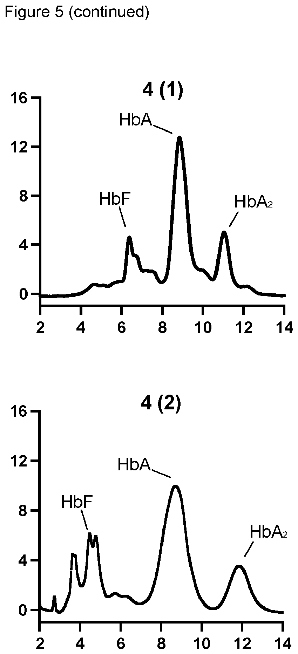

**Figure 5****. HPLC of homozygous knockin clones**
   Homozygous clones were isolated from editing conditions 2 (Cas9 + gRNA + DNA donor template containing only KLF1 binding site (KLF1 binding site having SEQ ID NO: 52; DNA donor template having SEQ ID NO: 64)), 3 (Cas9 + gRNA+ DNA donor template containing only the b-DRF motif and the TFIIB binding site (b-DRF motif having SEQ ID NO: 54 and TFIIB binding site having SEQ ID NO: 53; DNA donor template having SEQ ID NO: 65)) and 4 (Cas9 + gRNA+ DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55)) shown in Fig. 3.
   All haemoglobins are denoted in each graph indicating the peak that corresponds to HbF, HbA, and HbA2. These clones were differentiated and harvested for HPLC. The data is presented as a representative of two biological replicates.
   Y-axis shows mAU (mili absorbance units, also referred to as milli-arbitrary units). X-axis indicates elution time in minutes.

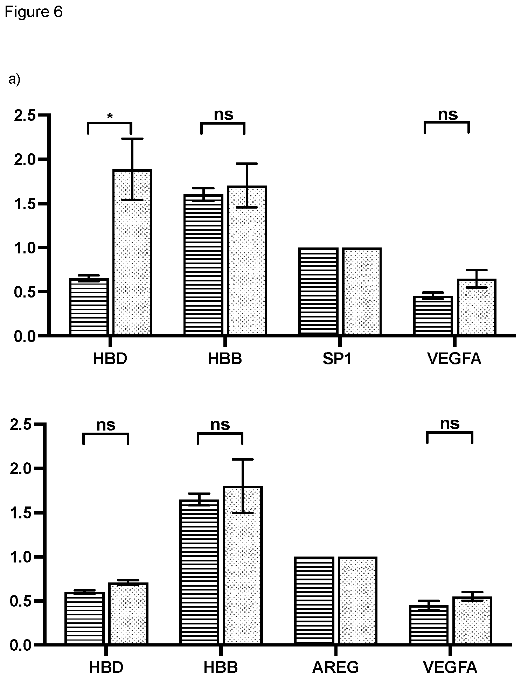

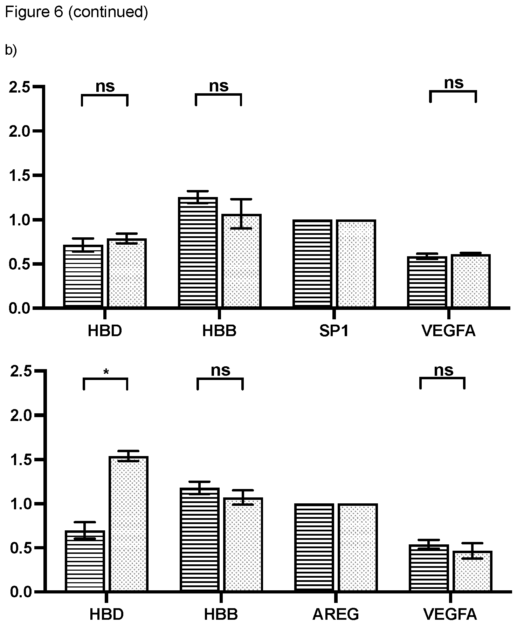

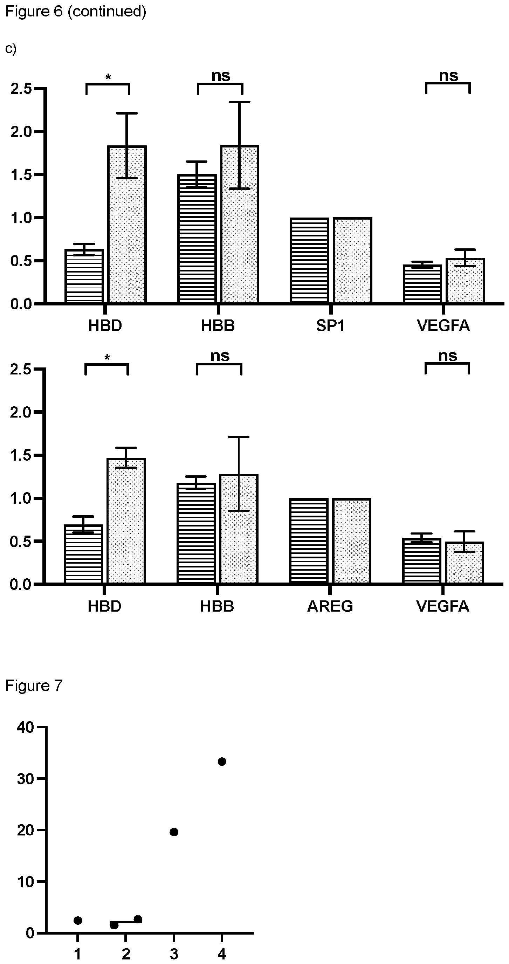

**Figure 6****. ChIP qPCR data for the homozygous knockin clones shows DNA-protein interaction of inserted transcription factor sequences.**
   Homozygous clones were isolated from editing conditions 2 (Cas9 + gRNA + DNA donor template containing only KLF1 binding site (KLF1 binding site having SEQ ID NO: 52; DNA donor template having SEQ ID NO: 64)), 3 (Cas9 + gRNA+ DNA donor template containing only the b-DRF motif and the TFIIB binding site (b-DRF motif having SEQ ID NO: 54 and TFIIB binding site having SEQ ID NO: 53; DNA donor template having SEQ ID NO: 65)) and 4 (Cas9 + gRNA + DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55)) shown in Fig. 3.
   Cells were harvested for ChIP of both KLF1 and TFIIB antibodies. Binding affinity was measured by ChIP-qPCR for inserted KLF1 and TFIIB motifs to further investigate protein-DNA interactions in edited clones. This demonstrates that the inserted sequences are bound by the KLF1 and TFIIB proteins. The data are presented as relative fraction of input and normalized to the positive control locus. The data is show as mean ± SD of three biological replicates. p value indicates paired, two-tailed Student's t test (ns, nonsignificant; *p % 0.05; **p % 0.01; ***p % 0.001).
      a) Investigation of KLF1 DNA interaction in the homozygous 2. KLF1 clone. Horizontal line bars indicate WT and dot bars indicate the homozygous clone. The assay was performed with a KLF1 antibody (top graph) and with a TFIIB antibody (bottom graph). The positive control locus for KLF1 is SP1 and negative locus is VEGFA. The positive control locus for TFIIB is AREG and the negative control locus is VEGFA.
      b) Investigation of TFIIB DNA interaction in the homozygous 3. b-DRF+TFIIB clone. Horizontal line bars indicate WT and dot bars indicate the homozygous clone. The assay was performed with a KLF1 antibody (top graph) and with a TFIIB antibody (bottom graph). The positive control locus for KLF1 is SP1 and negative locus is VEGFA. The positive control locus for TFIIB is AREG and the negative control locus is VEGFA.
      c) Investigation of TFIIB & KLF1 DNA interaction in the homozygous 4. KLF1+b-DRF+TFIIB clone. Horizontal line bars indicate WT and dot bars indicate the homozygous clone. The assay was performed with a KLF1 antibody (top graph) and with a TFIIB antibody (bottom graph). The positive control locus for KLF1 is SP1 and negative locus is VEGFA. The positive control locus for TFIIB is AREG and the negative control locus is VEGFA.
   Y-axis shows relative fraction of input.
**Figure 7****. qRT-PCR data (*HBD* expression) of edited CD34+ derived erythroblast colonies.**
   Individual colonies were isolated from editing CD34+ human HSPCs with (Cas9 + gRNA+ DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55))
   Colonies were grown under erythroid expansion conditions and were differentiated and harvested for qRT-PCR. Colonies were genotyped by NGS. The genotypes are as follows: 1 indicates a WT/NHEJ, 2 indicates NHEJ/NHEJ, 3 indicates NHEJ/HDR (heterozygous knock-in) and 4 indicates HDR/HDR (homozygous knock-in).
   Y-axis shows % HBD of beta-like globins. The beta-like globins measured include HBB, HBD, and HBG. HBD is shown as a percentage of the total of these globin genes.

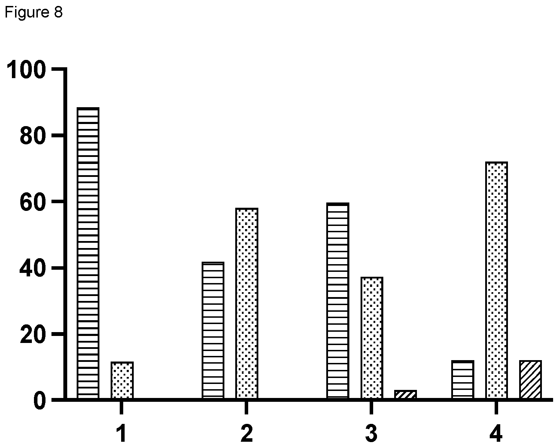

**Figure 8****. *HBD* gene editing efficiencies of several gRNAs**
   HUDEP-2 cells were edited with 2 different gRNAs and editing outcomes were compared.
   Each of these gRNA/Cas9 RNPs were tested both in the presence and absence of a DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55). gRNAs were used together with SpCas9 having SEQ ID NO: 56 (Cas9 RNP Nucleofection). The following conditions were used
      1. gRNA 1 (SEQ ID NO: 46; comprises DNA targeting segment having SEQ ID NO: 23) + Cas9 (no DNA donor template);
      2. gRNA 2 (SEQ ID NO: 47; comprises DNA targeting segment having SEQ ID NO: 26) + Cas9 (no DNA donor template);
      3. gRNA 1 (SEQ ID NO: 46; comprises DNA targeting segment having SEQ ID NO: 23) + Cas9 + DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55);
      4. gRNA 2 (SEQ ID NO: 47; comprises DNA targeting segment having SEQ ID NO: 26) + Cas9 + DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55).
   Cells were harvested for Sanger sequencing and Synthego ICE analysis was used to assign editing outcomes of percent unmodified, NHEJ, and HDR.
   The data is presented as one experimental replicate.
   Y-axis shows the % of editing outcomes as either unmodified, NHEJ, or HDR. Horizontal line bars indicate unmodified alleles, dot bars indicate NHEJ alleles, and slanted line bars indicate HDR alleles.

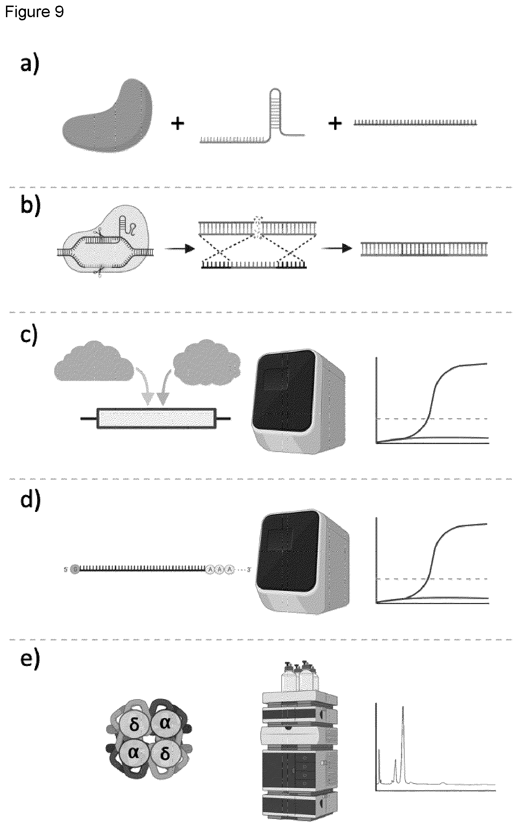

**Figure 9****. Overview of the experimental approach**
   a) Schematic representation of Cas9 polypeptide, gRNA and DNA donor template (inventive composition / kit)
   b) Cartoon showing editing of the human *HBD* gene using the inventive CRISPR/Cas composition/kit. Cas9 is guided by the gRNA to the promoter region of the human *HBD* gene and cuts the genomic DNA at a pre-determined location. A DNA donor template (single strand DNA shown; ssODN) serves as the repair template for *HDR* editing.
   c) Cartoon showing ChIP-qPCR measurement of binding activity of KLF1 or TFIIB at the edited region.
   d) Cartoon showing measurement of RNA level expression of HBD by qRT-PCR.
   e) Cartoon showing measurement of protein level expression of HbA2 by HPLC.

Surprisingly, it has been found that insertion of a human KLF1 binding site, a human b-DRF motif and a human TFIIB binding site in the promoter region of a human *HBD* gene leads to efficient expression of delta-globin in a therapeutically meaningful manner, thereby leading to increased expression of HbA2, even in the presence of a human *HBB* gene. This is contrary to previous findings suggesting that insertion of a KLF1 binding site alone is sufficient to drive expression of delta-globin in a therapeutically meaningful manner. Without wishing to be bound by theory, it is believed that competition between *HBB* and *HBD* genes for binding to KLF1 leads to insufficient expression of *HBD* when both genes are present and the *HBD* gene contains a KLF1 binding site as the sole modified transcription factor binding site in its promoter region.

In a first aspect, the invention relates to a CRISPR/Cas composition as defined below. This composition is suitable for editing a human *HBD* gene and therefore suitable for the treatment of haemoglobinopathies, such as anemia, particularly SCD or beta thalassemia.

It has been surprisingly found that the above defined technical problem is solved by a **CRISPR/Cas composition** comprising:
(a) a first component selected from
   (a-1) a **guide RNA** (gRNA) comprising a DNA targeting segment directed to the promoter region of a human *HBD* gene, preferably comprising a nucleotide sequence selected from the group consisting of SEQ ID NOs: 1 - 43, wherein the DNA targeting segment has a length of between 20 to 24 nucleotides, and
   (a-2) a **DNA polynucleotide encoding said gRNA,** optionally wherein said DNA polynucleotide encoding said gRNA is comprised in a vector, preferably an adeno-associated virus (AAV) vector or a lentiviral vector;
   (b) a second component selected from
      (b-1) a **DNA donor template,** and
      (b-2) a **vector,** preferably an AAV vector or a lentiviral vector, comprising the DNA donor template,
      wherein the DNA donor template comprises
      (b-i) a first homology arm sequence having at least 80% sequence identity to a first portion of a chromosomal DNA sequence comprised in the promoter region of the human *HBD* gene,
      (b-ii) a second homology arm sequence having at least 80% sequence identity to a second portion of a chromosomal DNA sequence comprised in the promoter region of the human *HBD* gene,
      (b-iii) a human KLF1 binding site, preferably having SEQ ID NO: 52,
      (b-iv) a human TFIIB binding site, preferably having SEQ ID NO: 53, and
      (b-v) a human b-DRF motif, preferably having SEQ ID NO: 54,
      wherein
      the first homology arm sequence and the second homology arm sequence independently of each other have a length of between 20 to 200 nucleotides, and the human KLF1 binding site, the human TFIIB binding site, and the human b-DRF motif are located 3' of the first homology arm sequence and 5' of the second homology arm sequence; and
   (c) a third component selected from
      (c-1) a **Cas9 polypeptide or a variant thereof,** and
      (c-2) a **nucleic acid encoding a Cas9 polypeptide or a variant thereof,** optionally comprised in a vector, preferably an AAV vector or a lentiviral vector.

As is common in the field, "directed to the promoter region of a human *HBD* gene" means that the DNA targeting segment of the gRNA comprises a contiguous stretch of between 20 to 24 nucleotides that are complementary to a target nucleic acid sequence within the human HBD promoter region.

Preferably, the first component is (a-1) the gRNA comprising a DNA targeting segment having a length of between 20 to 24 nucleotides and being directed to the promoter region of the human *HBD* gene.

Preferably, preferably the DNA targeting segment comprises a nucleotide sequence selected from the group consisting of SEQ ID NOs: 1 - 43.

Alternatively, the first component is (a-2) the DNA polynucleotide encoding the gRNA as defined above. In one embodiment, the DNA polynucleotide encoding the gRNA is comprised in a vector, preferably an adeno-associated virus (AAV) vector or a lentiviral vector.

Preferably, the second component is (b-1) the DNA donor template comprising:
(b-i) a first homology arm sequence having at least 80% sequence identity to a first portion of a chromosomal DNA sequence comprised in the promoter region of the human HBD gene,
(b-ii) a second homology arm sequence having at least 80% sequence identity to a second portion of a chromosomal DNA sequence comprised in the promoter region of the human HBD gene,
(b-iii) a human KLF1 binding site, preferably having SEQ ID NO: 52,
(b-iv) a human TFIIB binding site, preferably having SEQ ID NO: 53, and
(b-v) a human b-DRF motif, preferably having SEQ ID NO: 54,
wherein the first homology arm sequence and the second homology arm sequence independently of each other have a length of between 20 to 200 nucleotides, and the human KLF1 binding site, the human TFIIB binding site, and the human b-DRF motif are located 3' of the first homology arm sequence and 5' of the second homology arm sequence.

Preferably, the first homology arm sequence has at least 90% sequence identity, such as 95% sequence identity or 100% sequence identity to the first portion of the chromosomal DNA sequence comprised in the promoter region of the human HBD gene.

Preferably, the second homology arm sequence has at least 90% sequence identity, such as 95% sequence identity or 100% sequence identity to the second portion of the chromosomal DNA sequence comprised in the promoter region of the human HBD gene.

Alternatively, the second component is (b-2) the vector, preferably an AAV vector or a lentiviral vector, comprising the DNA donor template as defined above.

Preferably, the third component is (c-1) the Cas9 polypeptide or the variant thereof as described herein. Suitable Cas9 polypeptides and variants thereof include, but are not limited to, SpCas9, HiFi-Cas9, SpRY-Cas9 and Cpf1. Further details of the Cas9 polypeptide or the variant thereof are described below.

Alternatively, the third component is (c-2) a nucleic acid encoding the Cas9 polypeptide or the variant thereof as defined herein. In one embodiment, the nucleic acid encoding the Cas9 polypeptide or the variant thereof is comprised in a vector, preferably an AAV vector or a lentiviral vector.

In a preferred embodiment, the CRISPR/Cas composition comprises:
(a-1) the gRNA comprising a DNA targeting segment directed to the promoter region of a human HBD gene, preferably comprising a nucleotide sequence selected from the group consisting of SEQ ID NOs: 1 - 43, wherein the DNA targeting segment has a length of between 20 to 24 nucleotides, and
(b-1) the DNA donor template, wherein the DNA donor template comprises
   (b-i) a first homology arm sequence having at least 80% sequence identity to a first portion of a chromosomal DNA sequence comprised in the promoter region of the human HBD gene,
   (b-ii) a second homology arm sequence having at least 80% sequence identity to a second portion of a chromosomal DNA sequence comprised in the promoter region of the human HBD gene,
   (b-iii) a human KLF1 binding site, preferably having SEQ ID NO: 52,
   (b-iv) a human TFIIB binding site, preferably having SEQ ID NO: 53, and
   (b-v) a human b-DRF motif, preferably having SEQ ID NO: 54,
   wherein the first homology arm sequence and the second homology arm sequence independently of each other have a length of between 20 to 200 nucleotides, and the human KLF1 binding site, the human TFIIB binding site, and the human b-DRF motif are located 3' of the first homology arm sequence and 5' of the second homology arm sequence; and
(c-1) the Cas9 polypeptide or the variant thereof.

Based on the present disclosure, the skilled person understands that the CRISPR/Cas composition for editing a human HBD gene as defined above may contain any combination of the first, second and third components described herein (a-1 / a2, b-1 / b-2, and c-1 / c-2.

An advantage of increasing the expression of delta-globin from the *HBD* gene to thereby increase expression of HbA2 compared to increasing expression of HbF is that *HBD* is present in all erythrocytes.

As is known in the field, the genomic coordinates (GRCh38) of the human *HBD* gene are 11:5,232,837-5,234,482. The genomic coordinates of the human HBD promoter are 11:5,234,483-5,234,658.

In a preferred embodiment, (a) the gRNA or the DNA polynucleotide encoding said gRNA, (b) the DNA donor template or the vector comprising the DNA donor template sequence, and (c) the Cas9 polypeptide or the variant thereof, or the nucleic acid encoding said Cas9 polypeptide or the variant thereof are present in a cell, preferably a CD34+ hematopoietic stem cell, *in vitro* or ex *vivo.* Nevertheless, other cells are also possible, e.g. myeloid progenitor cells, erythroid progenitor cells and erythroblasts. The skilled person understands that a CD34+ hematopoietic stem cell may differentiate into myeloid progenitor cells, erythroid progenitor cells, erythroblasts and erythrocytes (in this order). Thus, the composition as described above may for example be present in a CD34+ hematopoietic stem cell that is then further differentiated into myeloid progenitor cells, erythroid progenitor cells, erythroblasts and/or erythrocytes. The skilled person is able to select a suitable cell type for editing a human *HBD* gene with the aim to increase HbA2 expression. However, it is to be understood that the cell is not a human germ cell. Differentiating CD34+ hematopoietic stem cells into myeloid progenitor cells, erythroid progenitor cells, erythroblasts and erythrocytes is within the ordinary skill.

This shall be explained in further detail below:
**CRISPR/Cas based genome editing:** CRISPR/Cas based genome editing is widely known in the field of biotechnology and generally refers to the process of modifying the nucleotide sequence of a genome using a CRISPR/Cas system, preferably in a precise or predetermined manner. In CRISPR/Cas based genome editing a Cas nuclease associates with a gRNA that directs the Cas nuclease towards a target sequence in chromosomal DNA. The Cas nuclease then typically binds to a protospacer adjacent motif (PAM) in the chromosomal DNA and subsequently leads to the cleavage of the DNA at a precise location.

Examples of methods of CRISPR/Cas based genome editing described herein include methods of using site-directed Cas nucleases, i.e. a Cas9 polypeptide or a variant thereof, to cut chromosomal DNA at precise target locations in the promoter region of a human *HBD* gene, thereby creating double-strand or single-strand DNA breaks at precise locations within the *HBD* gene promoter region. Such DNA breaks are generally repaired by natural, endogenous cellular processes such as homology-directed repair (HDR) and non-homologous end-joining (NHEJ).

In NHEJ, the DNA ends resulting from a double-strand break are directly joined together, sometimes leading to loss or addition of nucleotide sequence which may disrupt or enhance gene expression.

HDR utilizes a homologous sequence, or donor sequence, as a template for inserting a defined DNA sequence at the break point. Naturally, the homologous sequence is typically present in the endogenous genome, e.g. on a sister chromatid. Alternatively, the donor sequence may be an exogenous DNA, also referred to as a DNA donor template, such as a single-strand oligonucleotide or a duplex oligonucleotide, that has regions of high homology with the nuclease-cleaved locus, but which may also contain additional sequence elements or sequence changes such as deletions or insertions that can be incorporated into the cleaved target locus. The exogenous nucleic acid may also be a vector comprising said DNA donor template, such as a plasmid or a viral vector, e.g. an AAV vector or a lentiviral vector.

In the context of the present invention, HDR is preferred over NHEJ, since the human KLF1 binding site, the human TFIIB binding site, and the human b-DRF motif should be inserted in a pre-determined manner in the promoter region of the human *HBD* gene. The HDR/NHEJ ratio can be influenced towards a higher rate of HDR by administering to a cell, such as a CD34+ hematopoietic stem cell, a myeloid progenitor cell, an erythroid progenitor cell or an erythroblast, preferably a CD34+ hematopoietic stem cell, *in vitro* or ex *vivo* an exogenous DNA donor template as described herein. The skilled person is able to select a suitable cell type for editing a human *HBD* gene with the aim to increase HbA2 expression.

The skilled person further understands that DNA repair outcomes are generally balanced between NHEJ and HDR, and thus NHEJ can typically not be completely avoided.

Although HDR and NHEJ account for the majority of DNA break repairs, additional repair mechanisms exist, such as microhomology-mediated end joining (MMEJ), which may contribute to gene editing outcomes.

### First component / guide RNA:

The term "gRNA" is known in the field.

The CRISPR/Cas composition for editing a human *HBD* gene comprises at least one guide RNA (gRNA) or a DNA polynucleotide encoding said at least one gRNA.

The gRNA interacts with the Cas9 polypeptide (RNA-guided endonuclease) or variant thereof to direct the Cas9 polypeptide or variant thereof to a specific target site within the human *HBD* gene promoter region, at which site the DNA targeting segment located at the 5' end of the gRNA binds to a complementary region in the *HBD* gene promoter. The DNA targeting segment is typically also referred to as spacer sequence and the complementary region in the target gene is typically referred to as protospacer sequence.

As is known in the field, a gRNA typically comprises three regions:
- a first region at the 5' end that is complementary to the DNA target site (DNA targeting segment);
- a second internal region that typically forms a stem loop structure; and
- a third region at the 3' end that remains essentially single-stranded.

The DNA targeting segment of the gRNA thus determines the cleavage site at which the human *HBD* gene is cut by the Cas9 polypeptide or variant thereof.

In the present invention, DNA targeting segments with a length of between 20 to 24 nucleotides, such as 20, 21, 22, 23, or 24 nucleotides, have been found particularly beneficial.

Exemplary suitable DNA targeting segments are shown in Table 1 below. However, the skilled person understands that depending on the Cas9 polypeptide used, additional DNA targeting segments are also suitable. For example, SpRY-Cas9 as described below, does not require a specific PAM sequence for binding and thus the DNA targeting segment may comprise any contiguous stretch of 20 - 24 nucleotides length that is complementary to the human *HBD* gene promoter region.

**Table 1. Suitable DNA targeting segments including indication of targeted DNA strand and corresponding PAM sequence.**

| SEQ ID NO | DNA targeting segment 5' - 3' | Targeted DNA strand | Correspond-inq PAM |
|---|---|---|---|
| 1 | AACUGCUGAAAGAGAUGCGG | Plus strand | TGG |
| 2 | ACUGCUGAAAGAGAUGCGGU | Plus strand | GGG |
| 3 | CUGCUGAAAGAGAUGCGGUG | Plus strand | GGG |
| 4 | UGCGGUGGGGAGAUAUGUAG | Plus strand | AGG |
| 5 | GGAGAUAUGUAGAGGAGAAC | Plus strand | AGG |
| 6 | GAGAUAUGUAGAGGAGAACA | Plus strand | GGG |
| 7 | AUGACAGAACAGCCAAUCUC | Plus strand | AGG |
| 8 | UGACAGAACAGCCAAUCUCA | Plus strand | GGG |
| 9 | CCCUUAACUUGCCCUGAGAU | Minus strand | TGG |
| 10 | GCCAAUCUCAGGGCAAGUUA | Plus strand | AGG |
| 11 | CCAAUCUCAGGGCAAGUUAA | Plus strand | GGG |
| 12 | AGGGCAAGUUAAGGGAAUAG | Plus strand | TGG |
| 13 | UUAAGGGAAUAGUGGAAUGA | Plus strand | AGG |
| 14 | AGGUUGGUUUAAGAUAAGCA | Minus strand | GGG |
| 15 | CAGGUUGGUUUAAGAUAAGC | Minus strand | AGG |
| 16 | CCCUGCUCCAGUGAGCAGGU | Minus strand | TGG |
| 17 | UCUUAAACCAACCUGCUCAC | Plus strand | TGG |
| 18 | UCCUCCCUGCUCCAGUGAGC | Minus strand | AGG |
| 19 | ACCAACCUGCUCACUGGAGC | Plus strand | AGG |
| 20 | CCAACCUGCUCACUGGAGCA | Plus strand | GGG |
| 21 | ACCUGCUCACUGGAGCAGGG | Plus strand | AGG |
| 22 | CUCACUGGAGCAGGGAGGAC | Plus strand | AGG |
| 23 | CUCUGCCCUGCCUUUUAUGC | Minus strand | TGG |
| 24 | GAGGACAGGACCAGCAUAAA | Plus strand | AGG |
| 25 | ACAGGACCAGCAUAAAAGGC | Plus strand | AGG |
| 26 | CAGGACCAGCAUAAAAGGCA | Plus strand | GGG |
| 27 | UGCACCAUGGUGUCUGUUUG | Minus strand | AGG |
| 28 | GCAACCUCAAACAGACACCA | Plus strand | TGG |
| 29 | CUCAGGAGUCAGAUGCACCA | Minus strand | TGG |
| 30 | CAUGGUGCAUCUGACUCCUG | Plus strand | AGG |
| 31 | UUGACAGCAGUCUUCUCCUC | Minus strand | AGG |
| 32 | AGACUGCUGUCAAUGCCCUG | Plus strand | TGG |
| 33 | GACUGCUGUCAAUGCCCUGU | Plus strand | GGG |
| 34 | ACUGCUGUCAAUGCCCUGUG | Plus strand | GGG |
| 35 | CACGUUCACUUUGCCCCACA | Minus strand | GGG |
| 36 | CCACGUUCACUUUGCCCCAC | Minus strand | AGG |
| 37 | CCUGUGGGGCAAAGUGAACG | Plus strand | TGG |
| 38 | AAAGUGAACGUGGAUGCAGU | Plus strand | TGG |
| 39 | GUGAACGUGGAUGCAGUUGG | Plus strand | TGG |
| 40 | CGUGGAUGCAGUUGGUGGUG | Plus strand | AGG |
| 41 | UGCAGUUGGUGGUGAGGCCC | Plus strand | TGG |
| 42 | GCAGUUGGUGGUGAGGCCCU | Plus strand | GGG |
| 43 | UUGGUGGUGAGGCCCUGGGC | Plus strand | AGG |

Preferably, the DNA targeting segment is selected from SEQ ID NO: 23 and SEQ ID NO: 26, most preferably SEQ ID NO: 26.

The main function of the second and third region of the gRNA is to serve as a scaffold sequence (also referred to as universal sequence) necessary for binding to the Cas9 polypeptide or the variant thereof. The second internal region typically forms a stem loop structure, i.e. the second internal region typically forms a secondary structure comprising a stem and a loop, e.g. a hairpin. The length of the loop and the stem can vary. For example, the loop may range from 3 to 10 nucleotides in length, and the stem may range from 6 to 20 base pairs in length. The stem may also comprise one or more bulges with a length of 1 to 10 nucleotides. The overall length of the second region typically ranges from 20 to 60 nucleotides in length.

The guide RNA typically further comprises a third region at the 3' end that remains essentially single-stranded. The length of the third region can vary. Typically, the third region is more than 4 nucleotides in length. For example, the length of the third region can range from 5 to 60 nucleotides in length.

Thus the length of the scaffold region (i.e. combined length of the second and the third regions) of the gRNA may range from 30 to 120 nucleotides in length, typically from 70 to 90 nucleotides.

Selecting a suitable scaffold region is within the ordinary skill. An exemplary scaffold region is shown below (5'-3'): GUUUUAGAGCUAGAAATAGCAAGUUAAAAU-AAGGCUAGUCCGUUAUCAACUUGAAAAAGUGGCACCGAGUCGGUGCUUUUUU (SEQ ID NO: 44)

The skilled person understands that the gRNA may be a single molecule comprising all three regions or may consist of two separate molecules, typically referred to as crRNA and tracrRNA.

Preferably, the gRNA is a single molecule.

Several exemplary single molecule gRNAs (also referred to as sgRNA) are shown in table 2 below (SEQ ID NOs: 45 - 49). GRNAs having SEQ ID NOs: 46 (DNA targeting segment having SEQ ID NO: 23) and 47 (DNA targeting segment having SEQ ID NO: 26) were found particularly beneficial. Particularly preferred is the gRNA having SEQ ID NO: 47.

**Table 2. Exemplary gRNAs**

| SEQ ID NO | gRNA (5' - 3') |
|---|---|
| 45 | CAGGUUGGUUUAAGAUAAGCGUUUUAGAGCUAGAAATAGCAA GUUAAAAUAAGGCUAGUCCGUUAUCAACUUGAAAAAGUGGCA CCGAGUCGGUGCUUUUUU |
| 46 | CUCUGCCCUGCCUUUUAUGCGUUUUAGAGCUAGAAATAGCAA GUUAAAAUAAGGCUAGUCCGUUAUCAACUUGAAAAAGUGGCA CCGAGUCGGUGCUUUUUU |
| 47 | CAGGACCAGCAUAAAAGGCAGUUUUAGAGCUAGAAATAGCAA GUUAAAAUAAGGCUAGUCCGUUAUCAACUUGAAAAAGUGGCA CCGAGUCGGUGCUUUUUU |
| 48 | ACAGGACCAGCAUAAAAGGCGUUUUAGAGCUAGAAATAGCAA GUUAAAAUAAGGCUAGUCCGUUAUCAACUUGAAAAAGUGGCA CCGAGUCGGUGCUUUUUU |
| 49 | AGGUUGGUUUAAGAUAAGCAGUUUUAGAGCUAGAAATAGCAA GUUAAAAUAAGGCUAGUCCGUUAUCAACUUGAAAAAGUGGCA CCGAGUCGGUGCUUUUUU |

Alternatively, the gRNA consists of a crRNA and a tracrRNA. In this alternative embodiment, the crRNA contains the DNA targeting segment and one half of the "stem" of the second region of the gRNA, and the tracrRNA contains the other half of the "stem" of the second region of the gRNA and the third region of the gRNA.

Thus, in this embodiment, the crRNA and tracrRNA each contain a sequence of nucleotides that are complementary to one another. For example, the crRNA and the tracrRNA may each comprise a sequence of 6 to 20 nucleotides that base pairs to the other sequence to form a functional gRNA.

Provided the DNA targeting segment is given, selecting suitable crRNAs and tracrR-NAs that are suitable for editing a human *HBD* gene is within the ordinary skill.

In one embodiment, the gRNA may be provided as an RNA molecule. As is known in the field, the gRNA may be transcribed *in vitro* or, alternatively, may be chemically synthesized.

As is common in the field, the gRNA may also be chemically modified. Chemical modification of gRNA is routinely performed to increase their stability against nucleases, to increase their affinity for a target DNA and/or decrease the likelihood or degree of innate immune response.

A large number of suitable modifications are known in the art, particularly for chemically synthesized gRNAs. Typically, chemically modified gRNAs include one or more nucleotides modified at the 2' position of the sugar, particularly 2'-OMe, 2'-MOE, 2'-F modifications or mixtures thereof, and / or modification of the phosphate group, particularly phosphorothioate and/or phosphorodithioate modifications. Chemically synthesis of modified gRNAs is routinely performed by incorporation of nucleotide analogs during solid-phase synthesis.

Although fewer modifications are available for *in vitro* transcribed gRNAs, a number of nucleotide analogs can be routinely included by using the corresponding triphosphates of such nucleotide analogs, particularly pseudouridine (pseudouridine-5'-triphosphate) or N1-methyl-pseudouridine (N1-methyl-pseudouridine-5'-triphosphate), during *in vitro* transcription, e.g. using bacteriophage T7-RNA polymerase. Inclusion of pseudouridine or N1-methyl-pseudouridine increases nuclease stability and reduces activation of the innate immune system of *in vitro* transcribed (IVT) gRNAs.

The skilled person understands that the gRNA may also be provided as a DNA polynucleotide encoding said gRNA. In this embodiment, the DNA polynucleotide encoding the gRNA is typically comprised in a vector, preferably an AAV vector or a lentiviral vector. In this embodiment, the DNA polynucleotide encoding said gRNA is typically operably linked to a promoter sequence for expression of the gRNA, e.g. a promoter sequence that is recognized by RNA polymerase III, such as mammalian U6 or H1 promoters.

### Second component / DNA donor template:

The CRISPR/Cas composition for editing a human HBD gene further comprises a DNA donor template. As described above in the context of HDR, the DNA donor template contains regions of high homology (homology arms) with the DNA target site and further contains additional sequence elements that can be incorporated into the DNA target site, i.e. the promoter region of a human *HBD* gene.

The skilled person understands that the length of the DNA donor template can vary over a broad range, e.g. from 50 nucleotides to 5000 nucleotides such as from 100 nucleotides to 500 nucleotides.

Homology arms:
The main function of the homology arms is to permit homologous recombination of the donor template and the cleaved DNA target site within the human *HBD* gene promotor region using the endogenous HDR mechanism such that the human KLF1 binding site, the human b-DRF motif and the human TFIIB binding site (additional sequence elements described below) can be integrated into the chromosomal sequence.

A first homology arm sequence is located 5' of the additional sequence elements that are to be inserted into the human HBD gene promoter region and a second homology arm is located 3' of said additional sequence elements. Thus, said additional sequence elements are flanked by the first and the second homology arm sequences.

It has been found beneficial that the first and the second homology arm sequences, independently, have at least 80%, 81%, 82%, 83%, 84%, 85%, 86%, 87%, 88%, 89%, 90%, 91%, 92%, 93%, 94%, 95%, 96%, 97%, 98%, or 99% sequence identity with a sequence upstream or downstream to the cleavage site, i.e. a first portion and a second portion of a chromosomal DNA sequence comprised in the promoter region of the human *HBD* gene. For example, the first and the second homology arm sequences, independently, may have 95% or 100% sequence identity with chromosomal sequences upstream or downstream to the cleavage site.

Preferably, the first homology arm and the second homology arm, independently, have a length of between 20 to 200 nucleotides, more preferably of between 50 to 200 nucleotides.

A suitable first homology arm sequence is for example a DNA sequence having SEQ ID NO: 50 shown below or a DNA sequence containing a contiguous stretch of at least 20 nucleotides of SEQ ID NO: 50. The first homology arm sequence is located 5' of the additional sequence elements described below (i.e. human KLF1 binding site, human TFIIB binding site, and human b-DRF motif).

A suitable second homology arm sequence is for example a DNA sequence having SEQ ID NO: 51 shown below or a DNA sequence containing a contiguous stretch of at least 20 nucleotides of SEQ ID NO: 51.
SEQ ID NO: 50: 
ATTAGCCTAAAACACTTCTGCAAAAATGAAACTAGGAGGATATTTTTAGAAACAA
CTGCTGAAAGAGATGCGGTGGGGAGATATGTAGAGGAGAACAGGGTTTCTGAG
TCAAGACACACATGACAGAACAGCCAATCTCAGGGCAAGTTAAGGGAATAGTG
GAATGAAGGTTCATTTTTCATTCTCACAAACTAATGAA
SEQ ID NO: 51:

GCAGGGCAGAGTCGACTGTTGCTTACACTTTCTTCTGACATAACAGTGTTCACT
AGCAACCTCAAACAGACACCATGGTGCATCTGACTCCTGAGGAGAAGACTGCT
GTCAATGCCCTGTGGGGCAAAGTGAACGTGGATGCAGTTGGTGGTGAGGCCCT
GGGCAGGTTGGTATCAAGGTTATAAGAGAGGCTCAAGGAG

Additional sequence elements:
In the context of the present invention, the DNA donor template contains at least three additional sequence elements: a human KLF1 binding site, a human TFIIB binding site, and a human b-DRF motif.

Said additional sequence elements are inserted into the human *HBD* gene promoter region by homologous recombination as described above. Presence of the human KLF1 binding site, the human TFIIB binding site, and the human b-DRF motif in the edited human *HBD* gene permit efficient binding of the transcription factors KLF1 and TFIIB, thereby increasing expression of delta-globin from the human *HBD* gene in a therapeutically meaningful manner.

Preferably, the human KLF1 binding site has a DNA sequence having SEQ ID 52 shown below.

Preferably, the human TFIIB binding site has a DNA sequence having SEQ ID 53 shown below.

Preferably, the human b-DRF motif has a DNA sequence having SEQ ID 54 shown below.
SEQ ID NO: 52: CCACACCC (human KLF1 binding site)
SEQ ID NO: 53: GGGCTGG (human TFIIB binding site)
SEQ ID NO: 54: GAGGGCAGG (human b-DRF motif)

In one embodiment, the DNA donor template has SEQ ID NO: 55 shown below (human KLF1 binding site, human TFIIB binding site and human b-DRF motif are underlined). SEQ ID NO: 55:

GGAATAGTGGAATGAAGGTTCATTTTTCATTCTCACAAACTAATGAAACCCTGCT
TATCTTAAACCAACCTGCTCACCACACCCTGGAGCAGGGAGGGCAGGAGCCAG
GGCTGGGCATAAAAGGCAGGGCAGAGTCGACTGTTGCTTACACTTTCTTCTGA
CATAACAGTGTTCACTAGCAACCTCAAACAGAC

The DNA donor template can be linear or circular. In embodiments, the DNA donor template can be part of a vector, such as an AAV vector or a lentiviral vector.

### Third component / Cas9 polypeptide or variant thereof

Cas9 polypeptides or variants thereof are RNA-guided DNA endonucleases that are routinely used in the field of genome editing. Suitable Cas9 polypeptides or variants thereof comprise at least one nuclear localization signal, which permits entry of the Cas9 polypeptide or a variant thereof into the nuclei of mammalian cells, particularly CD34+ hematopoietic stem cell, *in vitro* or ex *vivo.* The skilled person understands that other cells are also possible, e.g. myeloid progenitor cells, erythroid progenitor cells and erythroblasts. Suitable Cas9 polypeptides or variants thereof further comprise at least one nuclease domain and at least one domain that interacts with a guide RNA. The Cas9 polypeptide or the variant thereof is directed to a specific nucleic acid sequence (or target site) within the human *HBD* gene promoter region by the gRNA. The gRNA interacts with both the Cas9 polypeptide or the variant thereof and the target site such that, once directed to the target site, the Cas9 polypeptide or the variant thereof is able to introduce a double-stranded break into the *HBD* gene promoter region. Since the gRNA provides the specificity for the targeted cleavage,
the Cas9 polypeptide or the variant thereof is universal and can be used with different gRNAs to cleave different target nucleic acid sequences. A broad range of Cas9 polypeptides or variants thereof are known in the field and are suitable for the present invention.

For example, the Cas9 polypeptide or the variant thereof can be from *Streptococcus pyogenes, Streptococcus thermophilus, Streptococcus sp.,Nocardiopsis dassonvillei, Streptomyces pristinaespiralis, Streptomyces viridochromogenes, Streptomyces viridochromogenes, Streptosporangium roseum, Streptosporangium roseum, Alicyclobacillus acidocaldarius, Bacillus pseudomycoides, Bacillus selenitireducens, Exiguobacterium sibiricum, Lactobacillus delbrueckii, Lactobacillus salivarius, Microscilla marina, Burkholderiales bacterium, Polaromonas naphthalenivorans, Polaromonas sp., Crocosphaera watsonii, Cyanothece sp., Microcystis aeruginosa, Synechococcus sp., Acetohalobium arabaticum, Ammonifex degensii, Caldicelulosiruptor becscii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooxidans, Allochromatium vinosum, Marinobactersp., Nitrosococcus halophilus, Nitrosococcus watsoni, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus, or Acaryochloris marina.*

The Cas9 polypeptide or the variant thereof can be a wild type Cas9 polypeptide, a modified Cas9 polypeptide, or a fragment of a wild type or modified Cas9 polypeptide. The Cas9 polypeptide or the variant thereof can be modified to increase nucleic acid binding affinity and/or specificity, alter an enzymatic activity, and/or change another property of the protein. Suitable modified Cas9 polypeptides or variants thereof are known in the art.

For example, nuclease (i.e., DNase, RNase) domains of the Cas9 polypeptide or the variant thereof can be modified, deleted, or inactivated. Such modified Cas9 polypeptides are commonly referred to as nickases.

Preferably, the Cas9 polypeptide or the variant thereof is selected from the group consisting of SpCas9, HiFi-Cas9, SpRY-Cas9 and Cpf1 (also referred to as Cas12a).

In one embodiment, the Cas9 polypeptide is SpCas9 having SEQ ID NO: 56 (corresponding DNA sequence is SEQ ID NO: 57). SpCas9 is a well-characterized Cas9 polypeptide (also considered the "original" Cas9) and was used in the examples shown below.

In another embodiment, the Cas9 polypeptide is HiFi-Cas9 having SEQ ID NO: 58 (corresponding DNA sequence is SEQ ID NO: 59). HiFi-Cas9 is a variant of a Cas9 polypeptide that has been mutated to reduce the number of off-target editing events. In another embodiment, the Cas9 polypeptide is SpRY-Cas9 having SEQ ID NO: 60 (corresponding DNA sequence is SEQ ID NO: 61). SpRY-Cas9 is a variant of a Cas9 polypeptide that has been mutated to recognize a broader range of PAM sequences. In another embodiment, the variant of the Cas9 polypeptide is Cpf1 having SEQ ID NO: 62 (corresponding DNA sequence is SEQ ID NO: 63). Cpf1 is a variant of a Cas9 polypeptide that produces staggered DNA cuts instead of blunt DNA cuts.

Sequences of Cas9-polypeptides and variants thereof:
SEQ ID NO: 56 (amino acid sequence of SpCas9):
SEQ ID NO: 57 (DNA sequence of SpCas9): 
ATGGACAAGAAGTACAGCATCGGCCTGGACATCGGCACCAACTCTGTGGGCTGGGCCGTGATCAC
CGACGAGTACAAGGTGCCCAGCAAGAAATTCAAGGTGCTGGGCAACACCGACCGGCACAGCATC
AAGAAGAACCTGATCGGAGCCCTGCTGTTCGACAGCGGCGAAACAGCCGAGGCCACCCGGCTGA
AGAGAACCGCCAGAAGAAGATACACCAGACGGAAGAACCGGATCTGCTATCTGCAAGAGATCTTC
AGCAACGAGATGGCCAAGGTGGACGACAGCTTCTTCCACAGACTGGAAGAGTCCTTCCTGGTGGA
AGAGGATAAGAAGCACGAGCGGCACCCCATCTTCGGCAACATCGTGGACGAGGTGGCCTACCAC
GAGAAGTACCCCACCATCTACCACCTGAGAAAGAAACTGGTGGACAGCACCGACAAGGCCGACCT
GCGGCTGATCTATCTGGCCCTGGCCCACATGATCAAGTTCCGGGGCCACTTCCTGATCGAGGGCG
ACCTGAACCCCGACAACAGCGACGTGGACAAGCTGTTCATCCAGCTGGTGCAGACCTACAACCAG
CTGTTCGAGGAAAACCCCATCAACGCCAGCGGCGTGGACGCCAAGGCCATCCTGTCTGCCAGACT
GAGCAAGAGCAGACGGCTGGAAAATCTGATCGCCCAGCTGCCCGGCGAGAAGAAGAATGGCCTG
TTCGGAAACCTGATTGCCCTGAGCCTGGGCCTGACCCCCAACTTCAAGAGCAACTTCGACCTGGC
CGAGGATGCCAAACTGCAGCTGAGCAAGGACACCTACGACGACGACCTGGACAACCTGCTGGCC
CAGATCGGCGACCAGTACGCCGACCTGTTTCTGGCCGCCAAGAACCTGTCCGACGCCATCCTGCT
GAGCGACATCCTGAGAGTGAACACCGAGATCACCAAGGCCCCCCTGAGCGCCTCTATGATCAAGA
GATACGACGAGCACCACCAGGACCTGACCCTGCTGAAAGCTCTCGTGCGGCAGCAGCTGCCTGA
GAAGTACAAAGAGATTTTCTTCGACCAGAGCAAGAACGGCTACGCCGGCTACATTGACGGCGGAG
CCAGCCAGGAAGAGTTCTACAAGTTCATCAAGCCCATCCTGGAAAAGATGGACGGCACCGAGGAA
CTGCTCGTGAAGCTGAACAGAGAGGACCTGCTGCGGAAGCAGCGGACCTTCGACAACGGCAGCA
TCCCCCACCAGATCCACCTGGGAGAGCTGCACGCCATTCTGCGGCGGCAGGAAGATTTTTACCCA
TTCCTGAAGGACAACCGGGAAAAGATCGAGAAGATCCTGACCTTCCGCATCCCCTACTACGTGGG
CCCTCTGGCCAGGGGAAACAGCAGATTCGCCTGGATGACCAGAAAGAGCGAGGAAACCATCACC
 
CCCTGGAACTTCGAGGAAGTGGTGGACAAGGGCGCTTCCGCCCAGAGCTTCATCGAGCGGATGA
CCAACTTCGATAAGAACCTGCCCAACGAGAAGGTGCTGCCCAAGCACAGCCTGCTGTACGAGTAC
TTCACCGTGTATAACGAGCTGACCAAAGTGAAATACGTGACCGAGGGAATGAGAAAGCCCGCCTT
CCTGAGCGGCGAGCAGAAAAAGGCCATCGTGGACCTGCTGTTCAAGACCAACCGGAAAGTGACC
GTGAAGCAGCTGAAAGAGGACTACTTCAAGAAAATCGAGTGCTTCGACTCCGTGGAAATCTCCGG
CGTGGAAGATCGGTTCAACGCCTCCCTGGGCACATACCACGATCTGCTGAAAATTATCAAGGACAA
GGACTTCCTGGACAATGAGGAAAACGAGGACATTCTGGAAGATATCGTGCTGACCCTGACACTGTT
TGAGGACAGAGAGATGATCGAGGAACGGCTGAAAACCTATGCCCACCTGTTCGACGACAAAGTGA
TGAAGCAGCTGAAGCGGCGGAGATACACCGGCTGGGGCAGGCTGAGCCGGAAGCTGATCAACG
GCATCCGGGACAAGCAGTCCGGCAAGACAATCCTGGATTTCCTGAAGTCCGACGGCTTCGCCAAC
AGAAACTTCATGCAGCTGATCCACGACGACAGCCTGACCTTTAAAGAGGACATCCAGAAAGCCCA
GGTGTCCGGCCAGGGCGATAGCCTGCACGAGCACATTGCCAATCTGGCCGGCAGCCCCGCCATT
AAGAAGGGCATCCTGCAGACAGTGAAGGTGGTGGACGAGCTCGTGAAAGTGATGGGCCGGCACA
AGCCCGAGAACATCGTGATCGAAATGGCCAGAGAGAACCAGACCACCCAGAAGGGACAGAAGAA
CAGCCGCGAGAGAATGAAGCGGATCGAAGAGGGCATCAAAGAGCTGGGCAGCCAGATCCTGAAA
GAACACCCCGTGGAAAACACCCAGCTGCAGAACGAGAAGCTGTACCTGTACTACCTGCAGAATGG
GCGGGATATGTACGTGGACCAGGAACTGGACATCAACCGGCTGTCCGACTACGATGTGGACCATA
TCGTGCCTCAGAGCTTTCTGAAGGACGACTCCATCGACAACAAGGTGCTGACCAGAAGCGACAAG
AACCGGGGCAAGAGCGACAACGTGCCCTCCGAAGAGGTCGTGAAGAAGATGAAGAACTACTGGC
GGCAGCTGCTGAACGCCAAGCTGATTACCCAGAGAAAGTTCGACAATCTGACCAAGGCCGAGAGA
GGCGGCCTGAGCGAACTGGATAAGGCCGGCTTCATCAAGAGACAGCTGGTGGAAACCCGGCAGA
TCACAAAGCACGTGGCACAGATCCTGGACTCCCGGATGAACACTAAGTACGACGAGAATGACAAG
CTGATCCGGGAAGTGAAAGTGATCACCCTGAAGTCCAAGCTGGTGTCCGATTTCCGGAAGGATTT
CCAGTTTTACAAAGTGCGCGAGATCAACAACTACCACCACGCCCACGACGCCTACCTGAACGCCG
TCGTGGGAACCGCCCTGATCAAAAAGTACCCTAAGCTGGAAAGCGAGTTCGTGTACGGCGACTAC
AAGGTGTACGACGTGCGGAAGATGATCGCCAAGAGCGAGCAGGAAATCGGCAAGGCTACCGCCA
AGTACTTCTTCTACAGCAACATCATGAACTTTTTCAAGACCGAGATTACCCTGGCCAACGGCGAGA
TCCGGAAGCGGCCTCTGATCGAGACAAACGGCGAAACCGGGGAGATCGTGTGGGATAAGGGCCG
GGATTTTGCCACCGTGCGGAAAGTGCTGAGCATGCCCCAAGTGAATATCGTGAAAAAGACCGAGG
TGCAGACAGGCGGCTTCAGCAAAGAGTCTATCCTGCCCAAGAGGAACAGCGATAAGCTGATCGCC
AGAAAGAAGGACTGGGACCCTAAGAAGTACGGCGGCTTCGACAGCCCCACCGTGGCCTATTCTGT
GCTGGTGGTGGCCAAAGTGGAAAAGGGCAAGTCCAAGAAACTGAAGAGTGTGAAAGAGCTGCTG
GGGATCACCATCATGGAAAGAAGCAGCTTCGAGAAGAATCCCATCGACTTTCTGGAAGCCAAGGG
CTACAAAGAAGTGAAAAAGGACCTGATCATCAAGCTGCCTAAGTACTCCCTGTTCGAGCTGGAAAA
CGGCCGGAAGAGAATGCTGGCCTCTGCCGGCGAACTGCAGAAGGGAAACGAACTGGCCCTGCCC
TCCAAATATGTGAACTTCCTGTACCTGGCCAGCCACTATGAGAAGCTGAAGGGCTCCCCCGAGGA
TAATGAGCAGAAACAGCTGTTTGTGGAACAGCACAAGCACTACCTGGACGAGATCATCGAGCAGA
TCAGCGAGTTCTCCAAGAGAGTGATCCTGGCCGACGCTAATCTGGACAAAGTGCTGTCCGCCTAC
AACAAGCACCGGGATAAGCCCATCAGAGAGCAGGCCGAGAATATCATCCACCTGTTTACCCTGAC
CAATCTGGGAGCCCCTGCCGCCTTCAAGTACTTTGACACCACCATCGACCGGAAGAGGTACACCA
GCACCAAAGAGGTGCTGGACGCCACCCTGATCCACCAGAGCATCACCGGCCTGTACGAGACACG
GATCGACCTGTCTCAGCTGGGAGGCGAC
SEQ ID NO: 58 (amino acid sequence of HiFi-Cas9): 
DKKYSIGLDIGTNSVGWAVITDEYKVPSKKFKVLGNTDRHSIKKNLIGALLFDSGETAEATRLKRTARRR
YTRRKNRICYLQEIFSNEMAKVDDSFFHRLEESFLVEEDKKHERHPIFGNIVDEVAYHEKYPTIYHLRKKL
VDSTDKADLRLIYLALAHMIKFRGHFLIEGDLNPDNSDVDKLFIQLVQTYNQLFEENPINASGVDAKAILS
ARLSKSRRLENLIAQLPGEKKNGLFGNLIALSLGLTPNFKSNFDLAEDAKLQLSKDTYDDDLDNLLAQIG
DQYADLFLAAKNLSDAILLSDILRVNTEITKAPLSASMIKRYDEHHQDLTLLKALVRQQLPEKYKEIFFDQS
KNGYAGYIDGGASQEEFYKFIKPILEKMDGTEELLVKLNREDLLRKQRTFDNGSIPHQIHLGELHAILRRQ
EDFYPFLKDNREKIEKILTFRIPYYVGPLARGNSRFAWMTRKSEETITPWNFEEVVDKGASAQSFIERMT
NFDKNLPNEKVLPKHSLLYEYFTVYNELTKVKYVTEGMRKPAFLSGEQKKAIVDLLFKTNRKVTVKQLKE
DYFKKIECFDSVEISGVEDRFNASLGTYHDLLKIIKDKDFLDNEENEDILEDIVLTLTLFEDREMIEERLKTY
AHLFDDKVMKQLKRRRYTGWGRLSRKLINGIRDKQSGKTILDFLKSDGFANANFMQLIHDDSLTFKEDI
QKAQVSGQGDSLHEHIANLAGSPAIKKGILQTVKVVDELVKVMGRHKPENIVIEMARENQTTQKGQKNS
RERMKRIEEGIKELGSQILKEHPVENTQLQNEKLYLYYLQNGRDMYVDQELDINRLSDYDVDHIVPQSFL
KDDSIDNKVLTRSDKNRGKSDNVPSEEVVKKMKNYWRQLLNAKLITQRKFDNLTKAERGGLSELDKAG
FIKRQLVETRQITKHVAQILDSRMNTKYDENDKLIREVKVITLKSKLVSDFRKDFQFYKVREINNYHHAHD
AYLNAVVGTALIKKYPKLESEFVYGDYKVYDVRKMIAKSEQEIGKATAKYFFYSNIMNFFKTEITLANGEI
RKRPLIETNGETGEIVWDKGRDFATVRKVLSMPQVNIVKKTEVQTGGFSKESILPKRNSDKLIARKKDW
DPKKYGGFDSPTVAYSVLVVAKVEKGKSKKLKSVKELLGITIMERSSFEKNPIDFLEAKGYKEVKKDLIIK
LPKYSLFELENGRKRMLASAGELQKGNELALPSKYVNFLYLASHYEKLKGSPEDNEQKQLFVEQHKHY
LDEIIEQISEFSKRVILADANLDKVLSAYNKHRDKPIREQAENIIHLFTLTNLGAPAAFKYFDTTIDRKRYTS
TKEVLDATLIHQSITGLYETRIDLSQLGGD
SEQ ID NO: 59 (DNA sequence of HiFi-Cas9): 
gacaagaagtacagcatcggcctggacatcggcaccaactctgtgggctgggccgtgatcaccgacgagtacaag
gtgcccagcaagaaattcaaggtgctgggcaacaccgaccggcacagcatcaagaagaacctgatcggagcgct
gctgttcgacagcggcgaaacagccgaggccacccggcttaagagaaccgccagaagaagatacaccagacg
gaagaaccggatctgctatctgcaagagatcttcagcaacgagatggccaaggtggacgacagcttcttccacaga
ctggaagagtccttcctggtggaagaggataagaagcacgagcggcaccccatcttcggcaacatcgtggacgag
gtggcctaccacgagaagtaccccaccatctaccacctgagaaagaaactggtggacagcaccgacaaggccga
cctgcggctgatctatctggccctggcccacatgatcaagttccggggccacttcctgatcgagggcgacctgaaccc
cgacaacagcgacgtggacaagctgttcatccagctggtgcagacctacaaccagctgttcgaggaaaaccccatc
aacgccagcggcgtggacgccaaggccatcctgtctgccagactgagcaagagcagacggctggaaaatctgat
cgcccagctgcccggcgagaagaagaatggcctgttcggaaacctgattgccctgagcctgggcctgacccccaa
cttcaagagcaacttcgacctggccgaggatgccaaactgcagctgagcaaggacacctacgacgacgacctgg
acaacctgctggcccagatcggcgaccagtacgccgacctgtttctggccgccaagaacctgtccgacgccatcctg
ctgagcgacatcctgagagtgaacaccgagatcaccaaggcccccctgagcgcctctatgatcaagagatacgac
gagcaccaccaggacctgaccctgctgaaagctctcgtgcggcagcagctgcctgagaagtacaaagagattttctt
cgaccagagcaagaacggctacgccggctacattgacggcggagccagccaggaagagttctacaagttcatca
agcccatcctggaaaagatggacggcaccgaggaactgctcgtgaagctgaacagagaggacctcctgaggaa
gcagcggaccttcgacaacggcagcatcccccaccagatccacctgggagagctgcacgccattctgcggcggca
ggaagatttttacccattcctgaaggacaaccgggaaaagatcgagaagatcctgaccttccgcatcccctactacgt
 
gggccctctggccaggggaaacagcagattcgcctggatgaccagaaagagcgaggaaaccatcaccccctgg
aacttcgaggaagtggtggacaagggcgcttccgcccagagcttcatcgagcggatgaccaacttcgataagaacc
tgcccaacgagaaggtgctgcccaagcacagcctgctgtacgagtacttcaccgtgtataacgagctgaccaaagt
gaaatacgtgaccgagggaatgagaaagcccgccttcctgagcggcgagcagaaaaaggccatcgtggacctgc
tgttcaagaccaaccggaaagtgaccgtgaagcagctgaaagaggactacttcaagaaaatcgagtgcttcgactc
cgtggaaatctccggcgtggaagatcggttcaacgcctccctgggcacataccacgatctgctgaaaattatcaagg
acaaggacttcctggacaatgaggaaaacgaggacattctggaagatatcgtgctgaccctgacactgtttgaggac
agagagatgatcgaggaacggctgaaaacctatgcccacctgttcgacgacaaagtgatgaagcagctgaagcg
gcggagatacaccggctggggcaggctgagccggaagctgatcaacggcatccgggacaagcagtccggcaag
acaatcctggatttcctgaagtccgacggcttcgccaacgccaacttcatgcaattgatccacgacgacagcctgacc
tttaaagaggacatccagaaagcccaggtgtccggccagggcgatagtctacacgagcacattgccaatctggccg
gcagccccgccattaagaagggcatcctgcagacagtgaaggtggtggacgagctcgtgaaagtgatgggccgg
cacaagcccgagaacatcgtgatcgaaatggccagagagaaccagaccacccagaagggacagaagaacag
ccgcgagagaatgaagcggatcgaagagggcatcaaagagctgggcagccagatcctgaaagaacaccccgt
ggaaaacacccagctgcagaacgagaagctgtacctgtactacctgcagaatgggcgggatatgtacgtggacca
ggaactggacatcaaccggctgtccgactacgatgtggaccatatcgtgcctcagagctttctgaaggacgactccat
cgacaacaaggtgctgaccagaagcgacaagaaccggggcaagagcgacaacgtgccctccgaagaggtcgt
gaagaagatgaagaactactggcggcagctgctgaacgccaagcttattacccagagaaagttcgacaatctgac
caaggccgagagaggcggcctgagcgaactggataaggccggcttcatcaagagacagctggtggaaacccgg
cagatcacaaagcacgtggcacagatcctggactcccggatgaacactaagtacgacgagaatgacaagctgatc
cgggaagtgaaagtgatcaccctgaagtccaagctggtgtccgatttccggaaggatttccagttttacaaagtgcgc
gagatcaacaactaccaccacgcccacgacgcctacctgaacgccgtcgtgggaaccgccctgatcaaaaagta
ccctaagctcgagagcgagttcgtgtacggcgactacaaggtgtacgacgtgcggaagatgatcgccaagagcga
gcaggaaatcggcaaggctaccgccaagtacttcttctacagcaacatcatgaactttttcaagaccgagattaccct
ggccaacggcgagatccggaagcggcctctgatcgagacaaacggcgaaaccggggagatcgtgtgggataag
ggccgggattttgccaccgtgcggaaagtgctgagcatgccccaagtgaatatcgtgaaaaagaccgaggtgcag
acaggcggcttcagcaaagagtctatcctgcccaagaggaacagcgataagctgatcgccagaaagaaggactg
ggaccctaagaagtacggcggcttcgacagccccaccgtggcctattctgtgctggtggtggccaaagtggaaaag
ggcaagtccaagaaactgaagagtgtgaaagagctgctggggatcaccatcatggaaagaagcagcttcgagaa
gaatcccatcgactttctggaagccaagggctacaaagaagtgaaaaaggacctgatcatcaagctgcctaagtac
tccctgttcgagctggaaaacggccggaagagaatgctggcctctgccggcgaactgcagaagggaaacgaactg
gccctgccctccaaatatgtgaacttcctgtacctggccagccactatgagaagctgaagggctcccccgaggataat
gagcagaaacagctgtttgtggaacagcacaagcactacctggacgagatcatcgagcagatcagcgagttctcca
agagagtgatcctggccgacgctaatctggacaaagtgctgtccgcctacaacaagcaccgggataagcccatca
gagagcaggccgagaatatcatccacctgtttaccctgaccaatctgggagcccctgccgccttcaagtactttgaca
ccaccatcgaccggaagaggtacaccagcaccaaagaggtgctggacgccaccctgatccaccagagcatcac
cggcctgtacgagacacggatcgacctgtctcagctgggaggcgac
SEQ ID NO: 60 (amino acid sequence of SpRY-Cas9): 
MDKKYSIGLDIGTNSVGWAVITDEYKVPSKKFKVLGNTDRHSIKKNLIGALLFDSGETAERTRLKRTARR
RYTRRKNRICYLQEIFSNEMAKVDDSFFHRLEESFLVEEDKKHERHPIFGNIVDEVAYHEKYPTIYHLRK
KLVDSTDKADLRLIYLALAHMIKFRGHFLIEGDLNPDNSDVDKLFIQLVQTYNQLFEENPINASGVDAKAIL
 
SARLSKSRRLENLIAQLPGEKKNGLFGNLIALSLGLTPNFKSNFDLAEDAKLQLSKDTYDDDLDNLLAQI
GDQYADLFLAAKNLSDAILLSDILRVNTEITKAPLSASMIKRYDEHHQDLTLLKALVRQQLPEKYKEIFFD
QSKNGYAGYIDGGASQEEFYKFIKPILEKMDGTEELLVKLNREDLLRKQRTFDNGSIPHQIHLGELHAILR
RQEDFYPFLKDNREKIEKILTFRIPYYVGPLARGNSRFAWMTRKSEETITPWNFEEVVDKGASAQSFIER
MTNFDKNLPNEKVLPKHSLLYEYFTVYNELTKVKYVTEGMRKPAFLSGEQKKAIVDLLFKTNRKVTVKQ
LKEDYFKKIECFDSVEISGVEDRFNASLGTYHDLLKIIKDKDFLDNEENEDILEDIVLTLTLFEDREMIEERL
KTYAHLFDDKVMKQLKRRRYTGWGRLSRKLINGIRDKQSGKTILDFLKSDGFANRNFMQLIHDDSLTFK
EDIQKAQVSGQGDSLHEHIANLAGSPAIKKGILQTVKVVDELVKVMGRHKPENIVIEMARENQTTQKGQ
KNSRERMKRIEEGIKELGSQILKEHPVENTQLQNEKLYLYYLQNGRDMYVDQELDINRLSDYDVDHIVP
QSFLKDDSIDNKVLTRSDKNRGKSDNVPSEEVVKKMKNYWRQLLNAKLITQRKFDNLTKAERGGLSEL
DKAGFIKRQLVETRQITKHVAQILDSRMNTKYDENDKLIREVKVITLKSKLVSDFRKDFQFYKVREINNYH
HAHDAYLNAVVGTALIKKYPKLESEFVYGDYKVYDVRKMIAKSEQEIGKATAKYFFYSNIMNFFKTEITLA
NGEIRKRPLIETNGETGEIVWDKGRDFATVRKVLSMPQVNIVKKTEVQTGGFSKESIRPKRNSDKLIARK
KDWDPKKYGGFLWPTVAYSVLVVAKVEKGKSKKLKSVKELLGITIMERSSFEKNPIDFLEAKGYKEVKK
DLIIKLPKYSLFELENGRKRMLASAKQLQKGNELALPSKYVNFLYLASHYEKLKGSPEDNEQKQLFVEQ
HKHYLDEIIEQISEFSKRVILADANLDKVLSAYNKHRDKPIREQAENIIHLFTLTRLGAPRAFKYFDTTIDPK
QYRSTKEVLDATLIHQSITGLYETRIDLSQLGGD
SEQ ID NO: 61 (DNA sequence of SpRY-Cas9): 
ATGGACAAGAAGTACAGCATCGGCCTGGACATCGGCACCAACTCTGTGGGCTGGGCCGTGATCAC
CGACGAGTACAAGGTGCCCAGCAAGAAATTCAAGGTGCTGGGCAACACCGACCGGCACAGCATC
AAGAAGAACCTGATCGGAGCCCTGCTGTTCGACAGCGGCGAAACAGCCGAGAGAACCCGGCTGA
AGAGAACCGCCAGAAGAAGATACACCAGACGGAAGAACCGGATCTGCTATCTGCAAGAGATCTTC
AGCAACGAGATGGCCAAGGTGGACGACAGCTTCTTCCACAGACTGGAAGAGTCCTTCCTGGTGGA
AGAGGATAAGAAGCACGAGCGGCACCCCATCTTCGGCAACATCGTGGACGAGGTGGCCTACCAC
GAGAAGTACCCCACCATCTACCACCTGAGAAAGAAACTGGTGGACAGCACCGACAAGGCCGACCT
GCGGCTGATCTATCTGGCCCTGGCCCACATGATCAAGTTCCGGGGCCACTTCCTGATCGAGGGCG
ACCTGAACCCCGACAACAGCGACGTGGACAAGCTGTTCATCCAGCTGGTGCAGACCTACAACCAG
CTGTTCGAGGAAAACCCCATCAACGCCAGCGGCGTGGACGCCAAGGCCATCCTGTCTGCCAGACT
GAGCAAGAGCAGACGGCTGGAAAATCTGATCGCCCAGCTGCCCGGCGAGAAGAAGAATGGCCTG
TTCGGAAACCTGATTGCCCTGAGCCTGGGCCTGACCCCCAACTTCAAGAGCAACTTCGACCTGGC
CGAGGATGCCAAACTGCAGCTGAGCAAGGACACCTACGACGACGACCTGGACAACCTGCTGGCC
CAGATCGGCGACCAGTACGCCGACCTGTTTCTGGCCGCCAAGAACCTGTCCGACGCCATCCTGCT
GAGCGACATCCTGAGAGTGAACACCGAGATCACCAAGGCCCCCCTGAGCGCCTCTATGATCAAGA
GATACGACGAGCACCACCAGGACCTGACCCTGCTGAAAGCTCTCGTGCGGCAGCAGCTGCCTGA
GAAGTACAAAGAGATTTTCTTCGACCAGAGCAAGAACGGCTACGCCGGCTACATTGACGGCGGAG
CCAGCCAGGAAGAGTTCTACAAGTTCATCAAGCCCATCCTGGAAAAGATGGACGGCACCGAGGAA
CTGCTCGTGAAGCTGAACAGAGAGGACCTGCTGCGGAAGCAGCGGACCTTCGACAACGGCAGCA
TCCCCCACCAGATCCACCTGGGAGAGCTGCACGCCATTCTGCGGCGGCAGGAAGATTTTTACCCA
TTCCTGAAGGACAACCGGGAAAAGATCGAGAAGATCCTGACCTTCCGCATCCCCTACTACGTGGG
CCCTCTGGCCAGGGGAAACAGCAGATTCGCCTGGATGACCAGAAAGAGCGAGGAAACCATCACC
CCCTGGAACTTCGAGGAAGTGGTGGACAAGGGCGCTTCCGCCCAGAGCTTCATCGAGCGGATGA
 
CCAACTTCGATAAGAACCTGCCCAACGAGAAGGTGCTGCCCAAGCACAGCCTGCTGTACGAGTAC
TTCACCGTGTATAACGAGCTGACCAAAGTGAAATACGTGACCGAGGGAATGAGAAAGCCCGCCTT
CCTGAGCGGCGAGCAGAAAAAGGCCATCGTGGACCTGCTGTTCAAGACCAACCGGAAAGTGACC
GTGAAGCAGCTGAAAGAGGACTACTTCAAGAAAATCGAGTGCTTCGACTCCGTGGAAATCTCCGG
CGTGGAAGATCGGTTCAACGCCTCCCTGGGCACATACCACGATCTGCTGAAAATTATCAAGGACAA
GGACTTCCTGGACAATGAGGAAAACGAGGACATTCTGGAAGATATCGTGCTGACCCTGACACTGTT
TGAGGACAGAGAGATGATCGAGGAACGGCTGAAAACCTATGCCCACCTGTTCGACGACAAAGTGA
TGAAGCAGCTGAAGCGGCGGAGATACACCGGCTGGGGCAGGCTGAGCCGGAAGCTGATCAACG
GCATCCGGGACAAGCAGTCCGGCAAGACAATCCTGGATTTCCTGAAGTCCGACGGCTTCGCCAAC
AGAAACTTCATGCAGCTGATCCACGACGACAGCCTGACCTTTAAAGAGGACATCCAGAAAGCCCA
GGTGTCCGGCCAGGGCGATAGCCTGCACGAGCACATTGCCAATCTGGCCGGCAGCCCCGCCATT
AAGAAGGGCATCCTGCAGACAGTGAAGGTGGTGGACGAGCTCGTGAAAGTGATGGGCCGGCACA
AGCCCGAGAACATCGTGATCGAAATGGCCAGAGAGAACCAGACCACCCAGAAGGGACAGAAGAA
CAGCCGCGAGAGAATGAAGCGGATCGAAGAGGGCATCAAAGAGCTGGGCAGCCAGATCCTGAAA
GAACACCCCGTGGAAAACACCCAGCTGCAGAACGAGAAGCTGTACCTGTACTACCTGCAGAATGG
GCGGGATATGTACGTGGACCAGGAACTGGACATCAACCGGCTGTCCGACTACGATGTGGACCATA
TCGTGCCTCAGAGCTTTCTGAAGGACGACTCCATCGACAACAAGGTGCTGACCAGAAGCGACAAG
AACCGGGGCAAGAGCGACAACGTGCCCTCCGAAGAGGTCGTGAAGAAGATGAAGAACTACTGGC
GGCAGCTGCTGAACGCCAAGCTGATTACCCAGAGAAAGTTCGACAATCTGACCAAGGCCGAGAGA
GGCGGCCTGAGCGAACTGGATAAGGCCGGCTTCATCAAGAGACAGCTGGTGGAAACCCGGCAGA
TCACAAAGCACGTGGCACAGATCCTGGACTCCCGGATGAACACTAAGTACGACGAGAATGACAAG
CTGATCCGGGAAGTGAAAGTGATCACCCTGAAGTCCAAGCTGGTGTCCGATTTCCGGAAGGATTT
CCAGTTTTACAAAGTGCGCGAGATCAACAACTACCACCACGCCCACGACGCCTACCTGAACGCCG
TCGTGGGAACCGCCCTGATCAAAAAGTACCCTAAGCTGGAAAGCGAGTTCGTGTACGGCGACTAC
AAGGTGTACGACGTGCGGAAGATGATCGCCAAGAGCGAGCAGGAAATCGGCAAGGCTACCGCCA
AGTACTTCTTCTACAGCAACATCATGAACTTTTTCAAGACCGAGATTACCCTGGCCAACGGCGAGA
TCCGGAAGCGGCCTCTGATCGAGACAAACGGCGAAACCGGGGAGATCGTGTGGGATAAGGGCCG
GGATTTTGCCACCGTGCGGAAAGTGCTGAGCATGCCCCAAGTGAATATCGTGAAAAAGACCGAGG
TGCAGACAGGCGGCTTCAGCAAAGAGTCTATCAGACCCAAGAGGAACAGCGATAAGCTGATCGCC
AGAAAGAAGGACTGGGACCCTAAGAAGTACGGCGGCTTCCTGTGGCCCACCGTGGCCTATTCTGT
GCTGGTGGTGGCCAAAGTGGAAAAGGGCAAGTCCAAGAAACTGAAGAGTGTGAAAGAGCTGCTG
GGGATCACCATCATGGAAAGAAGCAGCTTCGAGAAGAATCCCATCGACTTTCTGGAAGCCAAGGG
CTACAAAGAAGTGAAAAAGGACCTGATCATCAAGCTGCCTAAGTACTCCCTGTTCGAGCTGGAAAA
CGGCCGGAAGAGAATGCTGGCCTCTGCCAAGCAGCTGCAGAAGGGAAACGAACTGGCCCTGCCC
TCCAAATATGTGAACTTCCTGTACCTGGCCAGCCACTATGAGAAGCTGAAGGGCTCCCCCGAGGA
TAATGAGCAGAAACAGCTGTTTGTGGAACAGCACAAGCACTACCTGGACGAGATCATCGAGCAGA
TCAGCGAGTTCTCCAAGAGAGTGATCCTGGCCGACGCTAATCTGGACAAAGTGCTGTCCGCCTAC
AACAAGCACCGGGATAAGCCCATCAGAGAGCAGGCCGAGAATATCATCCACCTGTTTACCCTGAC
CAGACTGGGAGCCCCTAGAGCCTTCAAGTACTTTGACACCACCATCGACCCCAAGCAGTACAGAA
GCACCAAAGAGGTGCTGGACGCCACCCTGATCCACCAGAGCATCACCGGCCTGTACGAGACACG
GATCGACCTGTCTCAGCTGGGAGGCGAC
SEQ ID NO: 62 (amino acid sequence of Cpf1): 
MTQFEGFTNLYQVSKTLRFELIPQGKTLKHIQEQGFIEEDKARNDHYKELKPIIDRIYKTYADQCLQLVQL
DWENLSAAIDSYRKEKTEETRNALIEEQATYRNAIHDYFIGRTDNLTDAINKRHAEIYKGLFKAELFNGKV
LKQLGTVTTTEHENALLRSFDKFTTYFSGFYRNRKNVFSAEDISTAIPHRIVQDNFPKFKENCHIFTRLITA
VPSLREHFENVKKAIGIFVSTSIEEVFSFPFYNQLLTQTQIDLYNQLLGGISREAGTEKIKGLNEVLALAIQ
KNDETAHIIASLPHRFIPLFKQILSDRNTLSFILEEFKSDEEVIQSFCKYKTLLRNENVLETAEALFNELNSI
DLTHIFISHKKLETISSALCDHWDTLRNALYERRISELTGKITKSAKEKVQRSLKHEDINLQEIISAAGKELS
EAFKQKTSEILSHAHAALDQPLPTTLKKQEEKEILKSQLDSLLGLYHLLDWFAVDESNEVDPEFSARLTGI
KLEMEPSLSFYNKARNYATKKPYSVEKFKLNFQMPTLARGWDVNREKNNGAILFVKNGLYYLGIMPKQ
KGRYKALSFEPTEKTSEGFDKMYYDYFPDAAKMIPKCSTQLKAVTAHFQTHTTPILLSNNFIEPLEITKEI
YDLNNPEKEPKKFQTAYAKKTGDQKGYREALCKWIDFTRDFLSKYTKTTSIDLSSLRPSSQYKDLGEYY
AELNPLLYHISFQRIAEKEIMDAVETGKLYLFQIYNKDFAKGHHGKPNLHTLYWTGLFSPENLAKTSIKLN
GQAELFYRPKSRMKRMAHRLGEKMLNKKLKDQKTPIPDTLYQELYDYVNHRLSHDLSDEARALLPNVIT
KEVSHEIIKDRRFTSDKFFFHVPITLNYQAANSPSKFNQRVNAYLKEHPETPIIGIDRGERNLIYITVIDSTG
KILEQRSLNTIQQFDYQKKLDNREKERVAARQAWSVVGTIKDLKQGYLSQVIHEIVDLMIHYQAVVVLEN
LNFGFKSKRTGIAEKAVYQQFEKMLIDKLNCLVLKDYPAEKVGGVLNPYQLTDQFTSFAKMGTQSGFLF
YVPAPYTSKIDPLTGFVDPFVWKTIKNHESRKHFLEGFDFLHYDVKTGDFILHFKMNRNLSFQRGLPGF
MPAWDIVFEKNETQFDAKGTPFIAGKRIVPVIENHRFTGRYRDLYPANELIALLEEKGIVFRDGSNILPKLL
ENDDSHAIDTMVALIRSVLQMRNSNAATGEDYINSPVRDLNGVCFDSRFQNPEWPMDADANGAYHIAL
KGQLLLNHLKESKDLKLQNGISNQDWLAYIQELR
SEQ ID NO: 63 (DNA sequence of Cpf1):

ATGACACAGTTCGAGGGCTTTACCAACCTGTATCAGGTGAGCAAGACACTGCGGTTTGAGCTGATC
CCACAGGGCAAGACCCTGAAGCACATCCAGGAGCAGGGCTTCATCGAGGAGGACAAGGCCCGCA
ATGATCACTACAAGGAGCTGAAGCCCATCATCGATCGGATCTACAAGACCTATGCCGACCAGTGC
CTGCAGCTGGTGCAGCTGGATTGGGAGAACCTGAGCGCCGCCATCGACTCCTATAGAAAGGAGAA
AACCGAGGAGACAAGGAACGCCCTGATCGAGGAGCAGGCCACATATCGCAATGCCATCCACGACT
ACTTCATCGGCCGGACAGACAACCTGACCGATGCCATCAATAAGAGACACGCCGAGATCTACAAG
GGCCTGTTCAAGGCCGAGCTGTTTAATGGCAAGGTGCTGAAGCAGCTGGGCACCGTGACCACAAC
CGAGCACGAGAACGCCCTGCTGCGGAGCTTCGACAAGTTTACAACCTACTTCTCCGGCTTTTATAG
AAACAGGAAGAACGTGTTCAGCGCCGAGGATATCAGCACAGCCATCCCACACCGCATCGTGCAGG
ACAACTTCCCCAAGTTTAAGGAGAATTGTCACATCTTCACACGCCTGATCACCGCCGTGCCCAGCC
TGCGGGAGCACTTTGAGAACGTGAAGAAGGCCATCGGCATCTTCGTGAGCACCTCCATCGAGGAG
GTGTTTTCCTTCCCTTTTTATAACCAGCTGCTGACACAGACCCAGATCGACCTGTATAACCAGCTGC
TGGGAGGAATCTCTCGGGAGGCAGGCACCGAGAAGATCAAGGGCCTGAACGAGGTGCTGGCCCT
GGCCATCCAGAAGAATGATGAGACAGCCCACATCATCGCCTCCCTGCCACACAGATTCATCCCCC
TGTTTAAGCAGATCCTGTCCGATAGGAACACCCTGTCTTTCATCCTGGAGGAGTTTAAGAGCGACG
AGGAAGTGATCCAGTCCTTCTGCAAGTACAAGACACTGCTGAGAAACGAGAACGTGCTGGAGACA
GCCGAGGCCCTGTTTAACGAGCTGAACAGCATCGACCTGACACACATCTTCATCAGCCACAAGAA
GCTGGAGACAATCAGCAGCGCCCTGTGCGACCACTGGGATACACTGAGGAATGCCCTGTATGAGC
GGAGAATCTCCGAGCTGACAGGCAAGATCACCAAGTCTGCCAAGGAGAAGGTGCAGCGCAGCCT
GAAGCACGAGGATATCAACCTGCAGGAGATCATCTCTGCCGCAGGCAAGGAGCTGAGCGAGGCC

TTCAAGCAGAAAACCAGCGAGATCCTGTCCCACGCACACGCCGCCCTGGATCAGCCACTGCCTAC
AACCCTGAAGAAGCAGGAGGAGAAGGAGATCCTGAAGTCTCAGCTGGACAGCCTGCTGGGCCTG
TACCACCTGCTGGACTGGTTTGCCGTGGATGAGTCCAACGAGGTGGACCCCGAGTTCTCTGCCCG
GCTGACCGGCATCAAGCTGGAGATGGAGCCTTCTCTGAGCTTCTACAACAAGGCCAGAAATTATG
CCACCAAGAAGCCCTACTCCGTGGAGAAGTTCAAGCTGAACTTTCAGATGCCTACACTGGCCAGA
GGCTGGGACGTGAATAGAGAGAAGAACAATGGCGCCATCCTGTTTGTGAAGAACGGCCTGTACTA
TCTGGGCATCATGCCAAAGCAGAAGGGCAGGTATAAGGCCCTGAGCTTCGAGCCCACAGAGAAAA
CCAGCGAGGGCTTTGATAAGATGTACTATGACTACTTCCCTGATGCCGCCAAGATGATCCCAAAGT
GCAGCACCCAGCTGAAGGCCGTGACAGCCCACTTTCAGACCCACACAACCCCCATCCTGCTGTCC
AACAATTTCATCGAGCCTCTGGAGATCACAAAGGAGATCTACGACCTGAACAATCCTGAGAAGGAG
CCAAAGAAGTTTCAGACAGCCTACGCCAAGAAAACCGGCGACCAGAAGGGCTACAGAGAGGCCCT
GTGCAAGTGGATCGACTTCACAAGGGATTTTCTGTCCAAGTATACCAAGACAACCTCTATCGATCT
GTCTAGCCTGCGGCCATCCTCTCAGTATAAGGACCTGGGCGAGTACTATGCCGAGCTGAATCCCC
TGCTGTACCACATCAGCTTCCAGAGAATCGCCGAGAAGGAGATCATGGATGCCGTGGAGACAGGC
AAGCTGTACCTGTTCCAGATCTATAACAAGGACTTTGCCAAGGGCCACCACGGCAAGCCTAATCTG
CACACACTGTATTGGACCGGCCTGTTTTCTCCAGAGAACCTGGCCAAGACAAGCATCAAGCTGAAT
GGCCAGGCCGAGCTGTTCTACCGCCCTAAGTCCAGGATGAAGAGGATGGCACACCGGCTGGGAG
AGAAGATGCTGAACAAGAAGCTGAAGGATCAGAAAACCCCAATCCCCGACACCCTGTACCAGGAG
CTGTACGACTATGTGAATCACAGACTGTCCCACGACCTGTCTGATGAGGCCAGGGCCCTGCTGCC
CAACGTGATCACCAAGGAGGTGTCTCACGAGATCATCAAGGATAGGCGCTTTACCAGCGACAAGT
TCTTTTTCCACGTGCCTATCACACTGAACTATCAGGCCGCCAATTCCCCATCTAAGTTCAACCAGAG
GGTGAATGCCTACCTGAAGGAGCACCCCGAGACACCTATCATCGGCATCGATCGGGGCGAGAGA
AACCTGATCTATATCACAGTGATCGACTCCACCGGCAAGATCCTGGAGCAGCGGAGCCTGAACAC
CATCCAGCAGTTTGATTACCAGAAGAAGCTGGACAACAGGGAGAAGGAGAGGGTGGCAGCAAGG
CAGGCCTGGTCTGTGGTGGGCACAATCAAGGATCTGAAGCAGGGCTATCTGAGCCAGGTCATCCA
CGAGATCGTGGACCTGATGATCCACTACCAGGCCGTGGTGGTGCTGGAGAACCTGAATTTCGGCT
TTAAGAGCAAGAGGACCGGCATCGCCGAGAAGGCCGTGTACCAGCAGTTCGAGAAGATGCTGATC
GATAAGCTGAATTGCCTGGTGCTGAAGGACTATCCAGCAGAGAAAGTGGGAGGCGTGCTGAACCC
ATACCAGCTGACAGACCAGTTCACCTCCTTTGCCAAGATGGGCACCCAGTCTGGCTTCCTGTTTTA
CGTGCCTGCCCCATATACATCTAAGATCGATCCCCTGACCGGCTTCGTGGACCCCTTCGTGTGGA
AAACCATCAAGAATCACGAGAGCCGCAAGCACTTCCTGGAGGGCTTCGACTTTCTGCACTACGAC
GTGAAAACCGGCGACTTCATCCTGCACTTTAAGATGAACAGAAATCTGTCCTTCCAGAGGGGCCTG
CCCGGCTTTATGCCTGCATGGGATATCGTGTTCGAGAAGAACGAGACACAGTTTGACGCCAAGGG
CACCCCTTTCATCGCCGGCAAGAGAATCGTGCCAGTGATCGAGAATCACAGATTCACCGGCAGAT
ACCGGGACCTGTATCCTGCCAACGAGCTGATCGCCCTGCTGGAGGAGAAGGGCATCGTGTTCAG
GGATGGCTCCAACATCCTGCCAAAGCTGCTGGAGAATGACGATTCTCACGCCATCGACACCATGG
TGGCCCTGATCCGCAGCGTGCTGCAGATGCGGAACTCCAATGCCGCCACAGGCGAGGACTATAT
CAACAGCCCCGTGCGCGATCTGAATGGCGTGTGCTTCGACTCCCGGTTTCAGAACCCAGAGTGGC
CCATGGACGCCGATGCCAATGGCGCCTACCACATCGCCCTGAAGGGCCAGCTGCTGCTGAATCA
CCTGAAGGAGAGCAAGGATCTGAAGCTGCAGAACGGCATCTCCAATCAGGACTGGCTGGCCTACA
TCCAGGAGCTGCGCAAC

As is apparent to the skilled person based on the present disclosure, the third component may also be a nucleic acid encoding the Cas9 polypeptide as defined above or the variant thereof as defined above. The nucleic acid can be RNA or DNA.

In one embodiment, the nucleic acid encoding the Cas9 polypeptide or the variant thereof is mRNA. As is known in the field, the mRNA can be 5' capped and/or 3' polyadenylated. In another embodiment, the nucleic acid encoding the Cas9 polypeptide or the variant thereof is DNA. The DNA can be present in a vector, preferably an AAV vector or a lentiviral vector. In this embodiment, the DNA encoding the Cas9 polypeptide or the variant thereof is operably linked to a promoter sequence for expression in the mammalian cell of interest, particularly a CD34+ hematopoietic stem cell. The skilled person understands that other cells are also possible, e.g. myeloid progenitor cells, erythroid progenitor cells and erythroblasts.

As is known in the field, the nucleic acid encoding the Cas9 polypeptide or the variant thereof may be codon optimized for efficient translation into protein in the mammalian cell of interest, particularly a CD34+ hematopoietic stem cell. Suitable tools for codon optimisation are known in the field (e.g. from Integrated DNA Technologies; https://eu.idtdna.com/pages/tools/codon-optimization-tool).

Examples of suitable promoter sequences are CMV immediate-early promoter, SV40 promoter, RSV promoter, MMTV promoter, PGK promoter, and EF-1 promoter.

Each of the above mentioned components a, b and c of the CRISPR/Cas composition for editing a human *HBD* gene may be used separately from each other or in any possible combination.

Thus, in a **second aspect,** the invention relates to a CRISPR/Cas kit. This is suitable for editing a human *HBD* gene and therefore suitable for the treatment of haemoglobinopathies, such as anemia, particularly SCD or beta thalassemia.

The kit comprises:
(a) a first component selected from
   (a-1) a gRNA comprising a DNA targeting segment directed to the promoter region of a human *HBD* gene, preferably comprising a nucleotide sequence selected from the group consisting of SEQ ID NOs: 1 - 43, wherein the DNA targeting segment has a length of between 20 to 24 nucleotides, and
   (a-2) a DNA polynucleotide encoding said gRNA, optionally wherein said DNA polynucleotide encoding said gRNA is comprised in a vector, preferably an AAV vector or a lentiviral vector;
(b) a second component selected from
   (b-1) a DNA donor template, and
   (b-2) a vector, preferably an AAV vector or a lentiviral vector comprising the DNA donor template,
   wherein the DNA donor template comprises
   (b-i) a first homology arm sequence having at least 80% sequence identity to a first portion of a chromosomal DNA sequence comprised in the promoter region of the human *HBD* gene,
   (b-ii) a second homology arm sequence having at least 80% sequence identity to a second portion of a chromosomal DNA sequence comprised in the promoter region of the human *HBD* gene,
   (b-iii) a human KLF1 binding site, preferably having SEQ ID NO: 52,
   (b-iv) a human TFIIB binding site, preferably having SEQ ID NO: 53, and
   (b-v) a human b-DRF motif, preferably having SEQ ID NO: 54,
   wherein
   the first homology arm sequence and the second homology arm sequence independently of each other have a length of between 20 to 200 nucleotides, and the human KLF1 binding site, the human TFIIB binding site, and the human b-DRF motif are located 3' of the first homology arm sequence and 5' of the second homology arm sequence; and
(c) a third component selected from
   (c-1) a Cas9 polypeptide or a variant thereof, and
   (c-2) a nucleic acid encoding a Cas9 polypeptide or a variant thereof, optionally comprised in a vector, preferably an AAV vector or a lentiviral vector.

All components of the kit are as defined above in the context of the composition for editing a human *HBD* gene (first aspect).

As is common in the field, "directed to the promoter region of a human *HBD* gene" means that the DNA targeting segment of the gRNA comprises a contiguous stretch of between 20 to 24 nucleotides that are complementary to a target nucleic acid sequence within the human HBD promoter region.

Preferably, the first component is (a-1) the gRNA comprising a DNA targeting segment having a length of between 20 to 24 nucleotides and being directed to the promoter region of the human *HBD* gene.

Preferably, preferably the DNA targeting segment comprises a nucleotide sequence selected from the group consisting of SEQ ID NOs: 1 - 43.

Alternatively, the first component is (a-2) the DNA polynucleotide encoding the gRNA as defined above. In one embodiment, the DNA polynucleotide encoding the gRNA is comprised in a vector, preferably an adeno-associated virus (AAV) vector or a lentiviral vector.

Preferably, the second component is (b-1) the DNA donor template comprising:
(b-i) a first homology arm sequence having at least 80% sequence identity to a first portion of a chromosomal DNA sequence comprised in the promoter region of the human HBD gene,
(b-ii) a second homology arm sequence having at least 80% sequence identity to a second portion of a chromosomal DNA sequence comprised in the promoter region of the human *HBD* gene,
(b-iii) a human KLF1 binding site, preferably having SEQ ID NO: 52,
(b-iv) a human TFIIB binding site, preferably having SEQ ID NO: 53, and
(b-v) a human b-DRF motif, preferably having SEQ ID NO: 54,
wherein the first homology arm sequence and the second homology arm sequence independently of each other have a length of between 20 to 200 nucleotides, and the human KLF1 binding site, the human TFIIB binding site, and the human b-DRF motif are located 3' of the first homology arm sequence and 5' of the second homology arm sequence.

Preferably, the first homology arm sequence has at least 90% sequence identity, such as 95% sequence identity or 100% sequence identity to the first portion of the chromosomal DNA sequence comprised in the promoter region of the human HBD gene.

Preferably, the second homology arm sequence has at least 90% sequence identity, such as 95% sequence identity or 100% sequence identity to the second portion of the chromosomal DNA sequence comprised in the promoter region of the human HBD gene.

Alternatively, the second component is (b-2) the vector, preferably an AAV vector or a lentiviral vector, comprising the DNA donor template as defined above.

Preferably, the third component is (c-1) the Cas9 polypeptide or the variant thereof. Alternatively, the third component is (c-2) the nucleic acid encoding the Cas9 polypeptide or the variant thereof as defined herein. In one embodiment, the nucleic acid encoding the Cas9 polypeptide or the variant thereof is comprised in a vector, preferably an AAV vector or a lentiviral vector.

In a preferred embodiment, the CRISPR/Cas kit comprises:
(a-1) the gRNA comprising a DNA targeting segment directed to the promoter region of a human HBD gene, preferably comprising a nucleotide sequence selected from the group consisting of SEQ ID NOs: 1 - 43, wherein the DNA targeting segment has a length of between 20 to 24 nucleotides, and
(b-1) the DNA donor template, wherein the DNA donor template comprises
   (b-i) a first homology arm sequence having at least 80% sequence identity to a first portion of a chromosomal DNA sequence comprised in the promoter region of the human HBD gene,
   (b-ii) a second homology arm sequence having at least 80% sequence identity to a second portion of a chromosomal DNA sequence comprised in the promoter region of the human HBD gene,
   (b-iii) a human KLF1 binding site, preferably having SEQ ID NO: 52,
   (b-iv) a human TFIIB binding site, preferably having SEQ ID NO: 53, and
   (b-v) a human b-DRF motif, preferably having SEQ ID NO: 54,
   wherein the first homology arm sequence and the second homology arm sequence independently of each other have a length of between 20 to 200 nucleotides, and the human KLF1 binding site, the human TFIIB binding site, and the human b-DRF motif are located 3' of the first homology arm sequence and 5' of the second homology arm sequence; and
(c-1) the Cas9 polypeptide or the variant thereof.

Based on the present disclosure, the skilled person understands that the CRISPR/Cas kit as defined above is suitable for editing a human *HBD* gene and may contain any combination of the first, second and third component described herein (a-1 / a2, b-1 / b-2, and/or c-1 / c-2.

In a **third aspect,** the invention relates to a gRNA that is suitable for editing a human *HBD* gene. The gRNA is as defined above in the context of the composition for editing a human *HBD* gene (first aspect; first component). Based on the present disclosure, the skilled person understands that the gRNAs described herein are suitable for editing the promoter region of a human *HBD* gene in conjunction with any suitable Cas9 polypeptide or variant thereof as described herein. The skilled person further understands that the gRNAs described herein may also be used in conjunction with alternative DNA donor templates and thus permit the introduction of a broad range of nucleic acid sequences into the promoter region of a human *HBD* gene, such as transcription factor binding sites other than those described herein.

In a **fourth aspect,** the invention relates to DNA donor templates and to vectors comprising such DNA donor template that are suitable for editing a human *HBD* gene. Such vectors include AAV vectors and lentiviral vectors. The DNA donor template or the vector are as defined above in the context of the composition for editing a human *HBD* gene (first aspect; second component). Based on the present disclosure, the skilled person understands that the DNA donor templates or the vectors comprising the DNA donor template described herein are suitable for editing the promoter region of a human *HBD* gene. The skilled person further understands that the DNA donor templates or the vectors comprising the DNA donor template described herein may also be used in conjunction with gRNAs and / or Cas polypeptides other than those described herein.

In a **fifth aspect,** the invention relates to specific nucleic acids, such as vectors. Inventive nucleic acids according to this aspect comprise:
(A) a first nucleotide sequence encoding a gRNA comprising a DNA targeting segment directed to the promoter region of a human *HBD* gene, preferably comprising a nucleotide sequence selected from the group consisting of SEQ ID NOs: 1 - 43, wherein the DNA targeting segment has a length of between 20 to 24 nucleotides, wherein the first nucleotide sequence encoding said gRNA is operably linked to a promoter; and/or
(B) a second nucleotide sequence comprising a DNA donor template, wherein the DNA donor template comprises
   (B-i) a first homology arm sequence having at least 80% sequence identity to a first portion of a chromosomal DNA sequence comprised in the promoter region of a human HBD gene,
   (B-ii) a second homology arm sequence having at least 80% sequence identity to a second portion of a chromosomal DNA sequence comprised in the promoter region of a human HBD gene,
   (B-iii) a human KLF1 binding site, preferably having SEQ ID NO: 52,
   (B-iv) a human TFIIB binding site, preferably having SEQ ID NO: 53, and
   (B-v) a human b-DRF motif, preferably having SEQ ID NO: 54,
   wherein the first homology arm sequence and the second homology arm sequence each have a length of between 20 to 200 nucleotides, and the human KLF1 binding site, the human TFIIB binding site, and the human b-DRF motif are located 3' of the first homology arm sequence and 5' of the second homology arm sequence.

In one embodiment, the nucleic acid further comprises a third nucleotide sequence encoding a Cas9 polypeptide or a variant thereof as described herein, wherein the third nucleotide sequence is operably linked to a promoter as defined above in the context of the composition for editing a human *HBD* gene (first aspect, third component).

Preferably, the nucleic acid is a recombinant expression vector, preferably a viral vector, more preferably an AAV vector or a lentiviral vector.

In one embodiment, the nucleic acid contains:
(A) the first nucleotide sequence encoding a gRNA as defined above (first aspect of the invention; first component); and
(B) the second nucleotide sequence comprising a DNA donor template as defined above (first aspect of the invention, second component); and
(C) the third nucleotide sequence encoding a Cas9 polypeptide or a variant thereof as defined above (first aspect of the invention, third component).

In one embodiment, the nucleic acid contains:
(A) the first nucleotide sequence encoding a gRNA comprising as defined above (first aspect of the invention; first component); and
(C) the third nucleotide sequence encoding a Cas9 polypeptide or a variant thereof as defined above (first aspect of the invention, third component).

In this embodiment the nucleic acid does not contain (B) the second nucleotide sequence comprising a DNA donor template as defined above (first aspect of the invention, second component).

In one embodiment, the nucleic acid contains:
(A) the first nucleotide sequence encoding a gRNA as defined above (first aspect of the invention; first component); and
(B) the second nucleotide sequence comprising a DNA donor template as defined above (first aspect of the invention; second component).

In this embodiment, the nucleic acid does not contain (C) the third nucleotide sequence encoding a Cas9 polypeptide or a variant thereof as defined above (first aspect of the invention; third component).

In one embodiment, the nucleic acid contains:
(B) the second nucleotide sequence comprising a DNA donor template as defined above (first aspect of the invention; second component); and
(C) the third nucleotide sequence encoding a Cas9 polypeptide or a variant thereof as defined above (first aspect of the invention; third component).

In this embodiment, the nucleic acid does not contain (A) the first nucleotide sequence encoding a gRNA as defined above (first aspect of the invention; first component).

The skilled person understands that the materials described herein can be used in different formats and combinations. Thus, below are shown exemplary compositions and kits containing the materials described herein. However, this list is not exhaustive and the skilled person is able to vary the formats and the combination of the components described herein based on the present disclosure while still being inside the scope of the claims.

In one embodiment, the Cas9 polypeptide or the variant thereof and the gRNA are provided as DNA sequences as part of the same vector, e.g. the same plasmid or the same viral vector such as an AAV vector or lentiviral vector, and the DNA donor template is provided separately as a double-strand DNA.

In another embodiment, the Cas9 polypeptide or the variant thereof and the gRNA are provided as DNA sequences as part of the same vector, e.g. the same plasmid or the same viral vector such as an AAV vector or lentiviral vector, and the DNA donor template is provided separately as a single-strand DNA.

In another embodiment, the Cas9 polypeptide or the variant thereof and the gRNA are provided as DNA sequences as part of the same vector, e.g. the same plasmid or the same viral vector such as an AAV vector or lentiviral vector, and the DNA donor template is provided separately as part of a vector, e.g. a viral vector such as an AAV vector or a lentiviral vector.

In another embodiment, the Cas9 polypeptide or the variant thereof is provided as polypeptide (protein) and the gRNA is provided as RNA. Preferably, in this embodiment, the Cas9 polypeptide or the variant thereof and the gRNA are provided together as a ribonucleoprotein complex (RNP). In this embodiment, the DNA donor template is provided separately as a double-strand DNA.

In another embodiment, the Cas9 polypeptide or the variant thereof is provided as polypeptide (protein) and the gRNA is provided as RNA. Preferably, in this embodiment, the Cas9 polypeptide or the variant thereof and the gRNA are provided together as RNP. In this embodiment, the DNA donor template is provided separately as a single-strand DNA. This embodiment is shown in the examples described herein and is the most preferred embodiment.

In another embodiment, the Cas9 polypeptide or the variant thereof is provided as polypeptide (protein) and the gRNA is provided as RNA. Preferably, in this embodiment, the Cas9 polypeptide or the variant thereof and the gRNA are provided together as a ribonucleoprotein complex (RNP). In this embodiment, the DNA donor template is provided separately as part of a vector, e.g. a viral vector such as an AAV vector or a lentiviral vector.

In another embodiment, the Cas9 polypeptide or the variant thereof is provided as RNA (mRNA) and the gRNA is provided separately as RNA and the DNA donor template is provided separately as part of a vector, e.g. a viral vector such as an AAV vector or a lentiviral vector.

In another embodiment, the Cas9 polypeptide or the variant thereof is provided as RNA (mRNA) and the gRNA is provided separately as RNA and the DNA donor template is provided as a double-strand DNA.

In yet another embodiment, the Cas9 polypeptide or the variant thereof is provided as RNA (mRNA) and the gRNA is provided separately as RNA and the DNA donor template is provided as a single-strand DNA.

For example, in one embodiment the CRISPR/Cas composition or the kit for editing a human *HBD* gene comprises:
- the gRNA as defined above (first aspect of the invention; first component; a-1);
- the DNA donor template as defined above (first aspect of the invention, third component; b-1); and
- the Cas9 polypeptide or the variant thereof as defined above (first aspect of the invention, third component; c-1)

In another embodiment, the CRISPR/Cas composition or the kit for editing a human *HBD* gene comprises the nucleic acid containing:
- the first nucleotide sequence encoding a gRNA as defined above (first aspect of the invention; first component); and
- the third nucleotide sequence encoding a Cas9 polypeptide or a variant thereof as defined above (first aspect of the invention, third component).

In this embodiment CRISPR/Cas composition or the kit for editing a human *HBD* gene further comprises the DNA donor template as defined above (first aspect of the invention, second component).

In a sixth aspect, the invention relates to a method of manufacturing the gRNA as described herein.

As is known in the field, the gRNA as described herein may be chemically synthesized and can ben conveniently obtained from commercial suppliers such as AxoLabs, Synthego or Dharmacon.

However, obtaining chemically synthesized RNAs with a length of more than 60 nucleotides from commercial suppliers is relatively expansive. A relatively cheaper alternative is thus to commercially obtain the gRNA as a double-strand DNA and to produce the gRNA by *in vitro* transcription as is routinely performed in the field, e.g. using bacteriophage T7 polymerase.

The gRNA as described herein may thus be manufactured by a method comprising the steps:
- providing a double strand DNA comprising the same sequence as the gRNA operably linked to a promoter sequence, e.g. a T7 promoter, an RNA polymerase, such as T7-polymerase, ribonucleotidetriphosphates and a buffer adapted to the RNA polymerase
- annealing the double strand DNA, e.g. by heating to 95°C followed by cooling to 20°C;
- contacting the double strand DNA with the T7-polymerase, the ribonucleotidetriphosphates and the buffer; and
- performing *in vitro* transcription at a temperature adapted to the RNA polymerase, e.g. 37°C.

The skilled person understands that depending on the length and the specific sequence of the gRNA, the optimal reaction temperature and reaction time may vary. Typically, good results are obtained using the bacteriophage T7-polymerase at a temperature of 37°C for 5h.

In a **seventh aspect,** the invention relates to a method of editing a human *HBD* gene in a cell, e.g. a CD34+ hematopoietic stem cell, *in vitro* or ex *vivo.* The skilled person understands that other cells are also possible, e.g. myeloid progenitor cells, erythroid progenitor cells and erythroblasts. The method comprises the following steps:
Step 1: Introducing into the cell, simultaneously or successively, the gRNA or the DNA polynucleotide encoding the gRNA as described above (first aspect of the invention, first component; third aspect of the invention), the DNA donor template or the vector comprising the DNA donor template as described above (first aspect of the invention, second component; fourth aspect of the invention) and the Cas9 polypeptide or the variant thereof as described above (first aspect of the invention, third component).
Step 2: Culturing the cell.

In step 2, the gRNA directs the Cas9 polypeptide or the variant thereof to the promoter region of the human *HBD* gene, where the Cas9 polypeptide or the variant thereof introduces a double-stranded break, and the double-stranded break is repaired by a DNA repair process, particularly HDR, such that the human KLF1 binding site, the human TFIIB binding site and the human b-DRF motif present on the DNA donor template are integrated into the promoter region of the human *HBD* gene.

It is to be understood, that the method does not comprise a process for modifying the germ line genetic identity of a human being, i.e. the cell is not a human germ cell.

Preferably, the cell is a CD34+ hematopoietic stem cell, a myeloid progenitor cell, an erythroid progenitor cell, or an erythroblast, more preferably a CD34+ hematopoietic stem cell. CD34 is a well-established marker of human hematopoietic stem cells. Preferably, in step 1, gRNA and the Cas9 polypeptide or the variant thereof are introduced into the cell as an RNP.

As is apparent from the present disclosure, the compositions, kits and materials described herein offer a novel treatment option for haemoglobinopathies,
Thus, in an eighth aspect, the invention relates to the use of the CRISPR/Cas composition and the kit for editing a human *HBD* gene and the individual components described herein in medicine, particular the use in the treatment of a haemoglobinopathy, such as an anemia, more particularly the use in the treatment of sickle cell disease or beta-thalassemia.

The skilled person understands that the term "use in medicine" relates to the use as a medicament.

In certain embodiments, the CRISPR/Cas composition for editing a human *HBD* gene as described herein (particularly in the first aspect of the invention) is used in the treatment of a haemoglobinopathy, such as an anemia.

In one embodiment, the haemoglobinopathy is sickle cell disease.

In another embodiment, the haemoglobinopathy is beta thalassemia.

In certain embodiments, the CRISPR/Cas kit for editing a human *HBD* gene as described herein (particularly in the second aspect of the invention) is used in the treatment of a haemoglobinopathy, such as an anemia.

In one embodiment, the haemoglobinopathy is sickle cell disease.

In another embodiment, the haemoglobinopathy is beta thalassemia.

In certain embodiments, the gRNA or the DNA polynucleotide encoding the gRNA as described herein (particularly in the third aspect of the invention) is used in the treatment of a haemoglobinopathy, such as an anemia.

In one embodiment, the haemoglobinopathy is sickle cell disease.

In another embodiment, the haemoglobinopathy is beta thalassemia.

In certain embodiments, the DNA donor template or the vector comprising the DNA donor template as described herein (particularly in the fourth aspect of the invention) is used in the treatment of a haemoglobinopathy, such as an anemia.

In one embodiment, the haemoglobinopathy is sickle cell disease.

In another embodiment, the haemoglobinopathy is beta thalassemia.

In certain embodiments, the nucleic acids as described herein (particularly in the fifth aspect of the invention) are used in the treatment of a haemoglobinopathy, such as an anemia.

In one embodiment, the haemoglobinopathy is sickle cell disease.

In another embodiment, the haemoglobinopathy is beta thalassemia.

### Examples

To further illustrate the invention, the following **examples** are provided. These examples are provided with no intent to limit the scope of the invention.

### General experimental methods:

### HUDEP-2 cell culture and differentiation:

All cell culture was performed at 37°C in a humidified atmosphere containing 5% CO2. HUDEP-2 cells were cultured in a base medium of SFEM (Stemcell Technologies 9650) containing to a final concentration of dexamethasone 1uM (Sigma D4902-100MG), doxycycline 1ug/ml (Sigma D9891-1G), human stem cell factor 50ng/ml (PeproTech 300-07), erythropoietin 50ng/ml (Peprotech 100-64), and penstrept 1%. Cells were cultured at a density of 2e5 - 1e6 cells/ml. For differentiation, HUDEP-2 cells were centrifuged at 500g for 5 minutes, media was removed and replaced with differentiation media. Differentiation media consisted of a base media of IMDM+Glutamax (ThermoFisher 31980030) containing to a final concentration human serum 5% (Sigma H4522-100mL), heparin 2IU/ml (Sigma H3149-25KU), insulin 10ug/ml (Sigma I2643-25mg), erythropoietin 50ng/ml (Peprotech 100-64), holo-transferrin 500ug/ml (Sigma T0665-100mg), mifepristone 1uM (Sigma M8046-100MG), and doxycyline 1ug/ml (Sigma D9891-1G). Cells were differentiated for 5 days and then harvested for analysis.

### Cas9 RNP Nucleofection

In vitro transcribed (IVT) gRNAs were purified and complexed with purified SpCas9 polypeptide having SEQ ID NO: 56 with or without the addition of a DNA donor template. The nucleofection was performed using Lonza 4D-Nucleofector and using the P3 Primary Cell 96-well NucleofectorTM Kit (V4SP-3096) following manufacturer's instructions. The HUDEP-2 nucleofector code used was DD-100 and for primary HSPCs ER-100.

### IVT single molecule RNAs (sgRNA)

gRNA sequences were ordered as DNA oligonucleotides and formed into duplexes using a PCR thermocycler. The DNA template was transcribed to RNA using HiS-cribe^{™} T7 High Yield RNA Synthesis Kit (E2040S) following manufacturer protocol. The resulting RNA was purified using RNeasy Mini kit (74104) and Rnase-Free Dnasel Kit (79254).

### DNA donor templates

DNA donor templates were used as single strand DNA (ssODN). ssODNs having the sequences shown below were commercially obtained.

DNA donor template containing only human KLF1 binding site (SEQ ID NO 64; KLF1 binding site underlined; used for condition 2 in the following examples):

TTCATTTTTCATTCTCACAAACTAATGAAACCCTGCTTATCTTAAACCAACCTGCT
CACTGGAGCAGGGAGGACAGGACCAGCATAAAAGCCACACCCTGCAGGGCAG
AGTCGACTGTTGCTTACACTTTCTTCTGACATAACAGTGTTCACTAGCAACCTCA
AACAGACACCATGGTGCATCTGACTC

DNA donor template containing only the b-DRF motif and the TFIIB binding site (SEQ ID NO 65; human b-DRF and TFIIB binding sites underlined; used for condition 3 in the following examples):

GGAATAGTGGAATGAAGGTTCATTTTTCATTCTCACAAACTAATGAAACCCTGCT
TATCTTAAACCAACCTGCTCACTGGAGCAGGGAGGGCAGGAGCCAGGGCTGG
GCATAAAAGGCAGGGCAGAGTCGACTGTTGCTTACACTTTCTTCTGACATAACA
GTGTTCACTAGCAACCTCAAACAGAC

DNA donor template containing KLF1 and TFIIB binding sites and the b-DRF motif (SEQ ID NO 55; human b-DRF and TFIIB binding sites underlined; used for condition 4 in the following examples; inventive composition):

GGAATAGTGGAATGAAGGTTCATTTTTCATTCTCACAAACTAATGAAACCCTGCT
TATCTTAAACCAACCTGCTCACCACACCCTGGAGCAGGGAGGGCAGGAGCCAG
GGCTGGGCATAAAAGGCAGGGCAGAGTCGACTGTTGCTTACACTTTCTTCTGA
CATAACAGTGTTCACTAGCAACCTCAAACAGAC

### qRT-PCR

RNA was harvested from cells using Qiagen RNeasy Mini Kit and Rnase-Free Dnasel Kit following manufacturer's instructions. RNA was reverse transcribed to cDNA using Iscript^{™} Reverse Transcription Supermix (BioRad) and qRT-PCR reactions were set up using SsoAdvanced Universal SYBR Green or SsoFast^{™} EvaGreen Supermix (BioRad). Reactions were run on the StepOne Plus Real-Time PCR System (Applied Biosystems) or the QuantStudio 6 Flex (Thermo Fisher). Samples were analyzed using a two-step amplification and melt curves were obtained after 40 cycles. The Ct values for genes of interest were normalized to GAPDH, and expressions of genes are represented as 2-[ΔCt] or 2-[ΔΔCt] for fold change over control condition. All primers used for qRT-PCR are listed.

### qRT-PCR primer sequences:

GAPDH:
   Forward primer (SEQ ID NO: 66): CAACAGCGACACCCACTCCT
   Reverse primer (SEQ ID NO: 67): CACCCTGTTGCTGTAGCCAAA
HBA:
   Forward primer (SEQ ID NO: 68): GAGGCCCTGGAGAGGATGTTCC
   Reverse primer (SEQ ID NO: 69): ACAGCGCGTTGGGCATGTCGTC
HBB:
   Forward primer (SEQ ID NO: 70): TGTCCACTCCTGATGCTGTTATG
   Reverse primer (SEQ ID NO: 71): GGCACCGAGCACTTTCTTG
HBD:
   Forward primer (SEQ ID NO: 72): AACCTCAAGGGCACTTTTTCT
   Reverse primer (SEQ ID NO: 73): GGAAACAGTCCAGGATCTCAA
HBG:
   Forward primer (SEQ ID NO: 74): CCTGTCCTCTGCCTCTGCC
   Reverse primer (SEQ ID NO: 75): GGATTGCCAAAACGGTCAC

### Example 1: qRT-PCR of HBD expression after CRISPR/Cas9 RNP editing with DNA donor templates in HUDEP2 cells.

HUDEP2 cells were edited with ribonucleoprotein complexes (RNP) of SpCas9 (SEQ ID NO: 56) and gRNA in the presence of different DNA donor templates. Nucleofection was performed as described above. The gRNA used in this experiment had SEQ ID NO: 49, which comprises the DNA targeting segment having SEQ ID NO: 26. Conditions were as follows:
1. Cas9 + gRNA, no DNA donor templates
2. Cas9 + gRNA + DNA donor template containing only KLF1 binding site (KLF1 binding site having SEQ ID NO: 52; DNA donor template having SEQ ID NO: 64)
3. Cas9 + gRNA+ DNA donor template containing only the b-DRF motif and the TFIIB binding site (b-DRF motif having SEQ ID NO: 54 and TFIIB binding site having SEQ ID NO: 53; DNA donor template having SEQ ID NO: 65)
4. Cas9 + gRNA DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55).

Edited cells were nucleofected and differentiated for 5 days and then harvested for analysis.

HUDEP-2 cell culture and differentiation, Cas9 RNP Nucleofection, and *in vitro* transcription of gRNA was performed as described above.

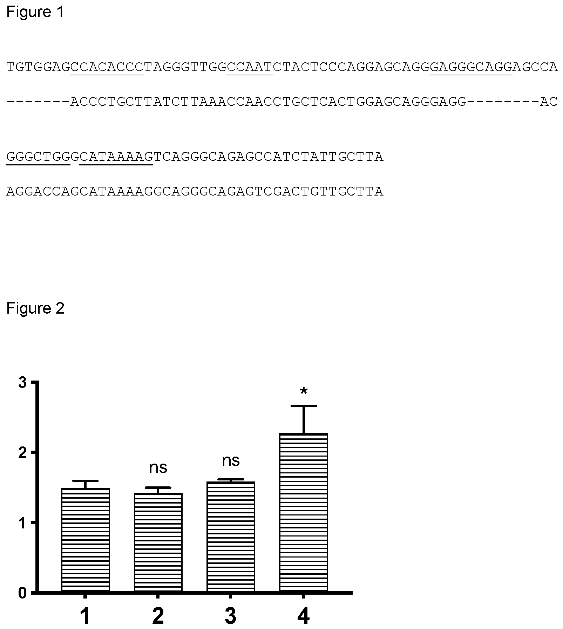

Results of this experiment are shown in Fig. 2.

This example shows that expression of human *HBD* gene is significantly increased using the inventive CRISPR/Cas composition (condition 4).

### Example 2: Analysis of editing outcomes in HUDEP2 cells

HUDEP2 cells were edited with Cas9 + gRNA RNP complexes with different DNA donor templates according to the general experimental methods described above. Conditions were as follows:
1. Cas9 + gRNA, no DNA donor templates
2. Cas9 + gRNA + DNA donor template containing only KLF1 binding site (KLF1 binding site having SEQ ID NO: 52; DNA donor template having SEQ ID NO: 64)
3. Cas9 + gRNA+ DNA donor template containing only the b-DRF motif and the TFIIB binding site (b-DRF motif having SEQ ID NO: 54 and TFIIB binding site having SEQ ID NO: 53; DNA donor template having SEQ ID NO: 65)
4. Cas9 + gRNA+ DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55).

To evaluate editing outcomes, samples were analysed by next-generation sequencing (NGS) as described below:
To confirm homozygosity/heterozygosity of the HBD clones, samples' genomic DNA extracted with QuickExtract DNA Kit was first amplified by PCR. The primers were designed specifically for NGS, spanning a region of < 200 bp (including the primers sequences) in which the cutsite is asymmetrically placed (e.g., 30-80 bp from the forward or the reverse primer) to capture the edited region. Subsequently, two stubber sequences were added, one for the forward primer (5'- CTTTCCCTACACGAC-GCTCTTCCGATCT -3'; SEQ ID NO: 76) and one for the reverse primer (5'- GGAG-TTCAGACGTGTGCTCTTCCGATCT -3'; SEQ ID NO: 77). After running the first PCR to amplify the genetic region of interest, the overhanging stubber sequences were used to run a second PCR with indexing primers (forward and reverse primers were premixed at 5 M). Samples were pooled and purified with SPRIselect beads, 5 mL (Beckman Coulter, B23317). In details, 1 volume of SPRI beads was added to the pooled PCRs, and the mix was incubated at room temperature for 1 minute. The reaction vessel was placed on a DynaMag^{™}-2 Magnet magnetic stand (Thermo Fisher Scientific, 12321D) to allow the beads to settle to the magnet, while the supernatant was discarded. With the reaction tubes still on the magnet, 180 ul of 85% EtOH were added to briefly wash the beads. After letting the beads drying for 10 minutes at room temperature, the reaction vessel was removed from the magnetic stand, and the appropriate volume of Elution Buffer EB (QIAGEN, 19086) was added to the beads and mixed by pipetting. The tubes were placed again on the magnetic stands to allow the SPRI beads to settle to the magnet, and the supernatant was collected and transferred into a new tube. The DNA samples were normalized for NGS submission, 20 ul at 10 nM concentration. NGS was performed by the Genome Engineering and Measurement Lab (geml, ETH Zürich) using a NovaSeq 6000 Sequencing System (Illumina, 20012850). Sequencing mode used was 10 PE (paired end), with 2x 8 bp barcode reads.

The following NGS primers were used in this experiment:
Primer pair 1 (SP21_fwd, SP22_rev)
   Forward primer (SEQ ID NO: 78): CACACATGACAGAACAGCCAATCTCAG
   Reverse primer (SEQ ID NO: 79): GAAGAAAGTGTAAGCAACAGTCGACTCTG
Primer pair 2 (SP23_fwd, SP24_rev)
   Forward primer (SEQ ID NO: 80): GGGCAAGTTAAGGGAATAGTGGAATGAAGG
   Reverse primer (SEQ ID NO: 81): GAGGTTGCTAGTGAACACTGTTATGTCAGAAG

HUDEP-2 cell culture and differentiation, Cas9 RNP Nucleofection, and *in vitro* transcription of gRNA was performed as described above.

The results of this experiment are shown in Fig. 3.

This example shows that the inventive CRISPR/Cas composition (condition 4) can be used to efficiently edit a human *HBD* gene via HDR.

### Example 3. qRT-PCR of heterozygous and homozygous knockin clones.

Heterozygous and homozygous clones from example 2 were isolated from editing conditions 2 (Cas9 + gRNA + DNA donor template containing only KLF1 binding site (KLF1 binding site having SEQ ID NO: 52; DNA donor template having SEQ ID NO:
64)), 3 (Cas9 + gRNA+ DNA donor template containing only the b-DRF motif and the TFIIB binding site (b-DRF motif having SEQ ID NO: 54 and TFIIB binding site having SEQ ID NO: 53; DNA donor template having SEQ ID NO: 65)) and 4 (Cas9 + gRNA+ DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55)).

These clones were differentiated and harvested for qRT-PCR according to the protocol described above. HUDEP-2 cell culture and differentiation, Cas9 RNP Nucleofection, and in vitro transcription of gRNA was performed as described above.

Results of this experiment are shown in Fig. 4. For condition 4, two homozygous clones were isolated, denoted by 4(1) and 4(2).

This example shows that expression of human *HBD* gene is significantly increased after genome editing using the inventive CRISPR/Cas composition (condition 4).

### Example 4. HPLC of homozygous knockin clones

Homozygous clones from example 2 were isolated from editing conditions 2 (Cas9 + gRNA + DNA donor template containing only KLF1 binding site (KLF1 binding site having SEQ ID NO: 52; DNA donor template having SEQ ID NO: 64)), 3 (Cas9 + gRNA+ DNA donor template containing only the b-DRF motif and the TFIIB binding site (b-DRF motif having SEQ ID NO: 54 and TFIIB binding site having SEQ ID NO: 53; DNA donor template having SEQ ID NO: 65)) and 4 (Cas9 + gRNA+ DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55)).

HUDEP-2 cells were differentiated and harvested for lysis in hemolysate reagent containing 0.005M EDTA and 0.07% KCN at 10,000 cells per microliter. The lysis was incubated at room temperature for ten minutes and then centrifuged at max speed for 5 minutes. The supernatant was collected and run on Agilent 1260 Infinity II using a PolyCAT A column, 35x4.6mm (3µm;1500Å) Serial# B19916E; Lot# 16-133-3 3.54CT0315. The following Buffer compositions were used: Mobile Phase A: 20mM Bis-tris, 2mM NaCN pH 6.8 and Mobile Phase B: 20mM Bis-tris, 2mM NaCN, 200mM NaCl, pH 6.9. The following flow settings were used: Gradient: 0-8' 2-25% Phase B, 8-18' 25-100% Phase B, 18-23' 100-2% Mobile Phase B using a Flow Rate: 1.5mL/min and measuring detection of 415nm Diode Array.

Results of this experiment are shown in Fig. 5 (HPLC traces). Areas under the HPLC curves were measured in order to assign percentages of haemoglobins HbF, HbA, and HbA2 results are shown in table 3 below.

**Table 3. Quantified percentages of total haemoglobins in homozygous knockin clones**

| Hemoglobin Percentage % | | | |
|---|---|---|---|
| Clone | HbF | HbA | HbA2 |
| WT | 14.4 | 84.3 | 1.3 |
| 2 | 11.5 | 83.9 | 4.6 |
| 3 | 22.1 | 74.7 | 3.2 |
| 4(1) | 17.5 | 60.6 | 21.9 |
| 4(2) | 20.4 | 60.1 | 19.5 |

This example shows that protein levels of HbA2 are significantly increased using the inventive CRISPR/Cas composition (condition 4).

### Example 5. ChIP-qPCR for homozygous knockin clones from example 2 shows DNA-protein interaction of inserted transcription factor sequences.

Homozygous clones were isolated from example 2 from editing conditions 2 (Cas9 + gRNA + DNA donor template containing only KLF1 binding site (KLF1 binding site having SEQ ID NO: 52; DNA donor template having SEQ ID NO: 64)), 3 (Cas9 + gRNA+ DNA donor template containing only the b-DRF motif and the TFIIB binding site (b-DRF motif having SEQ ID NO: 54 and TFIIB binding site having SEQ ID NO: 53; DNA donor template having SEQ ID NO: 65)) and 4 (Cas9 + gRNA+ DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55)).

Cells were harvested for ChIP of both KLF1 and TFIIB antibodies. Binding affinity was measured by ChIP-qPCR for inserted KLF1 and TFIIB transcription factors to further investigate protein-DNA interactions in edited clones as described below:
10 million cells per sample were harvested and cross-linked in 1% Formaldehyde. Cross-linking was quenched with the addition of 1.5M glycine. Samples were then lysed for 10 minutes at 4C in 50 mM Hepes-KOH, pH 7.5; 140 mM NaCl; 1 mM EDTA; 10% glycerol; 0.5% NP-40 or Igepal CA-630; 0.25% Triton X-100. Cells were then centrifuged at 1500g for 3 minutes and the supernatant was discarded. The pellet was resuspended in 10 mM Tris-HCl, pH8.0; 200 mM NaCl; 1 mM EDTA; 0.5 mM EGTA and incubated for 5 minutes at 4C. The cells were then centrifuged at 1500g for 3 minutes and the supernatant was discarded. The pellet was resuspended in 10 mM Tris-HCl, pH 8; 100 mM NaCl; 1 mM EDTA; 0.5 mM EGTA; 0.1% Na-Deoxycholate; 0.5% N-lauroylsarcosine and sonicated using the Covaris S220 following manufacturer's instructions. Protein A beads (ThermoFisher) were complexed with antibody and the antibody-bead complexes were incubated with cell lysates at 4C overnight with rotation. The antibodies used were rabbit anti-ATF4 (CST 11815S) and rabbit IgG (Novus Biologicals NBP2-24891). The beads were retrieved using a magnetic stand and rinsed with RIPA buffer. Elution buffer containing 50 mM Tris-HCl, pH 8; 10 mM EDTA; 1% SDS was added to the beads for reverse crosslinking at 65C overnight with shaking. After reverse crosslinking, the beads were removed. The eluted DNA was treated with RNaseA and Proteinase K and then purified using Qiagen MinElute PCR Purification Kit, following the manufacturer's instructions. Q-PCR reactions were set up using SsoAdvanced Universal SYBR Green or SsoFast^{™} EvaGreen Supermix (BioRad). Reactions were run on the StepOne Plus Real-Time PCR System (Applied Biosystems) or the QuantStudio 6 Flex (Thermo Fisher). The Ct values were analyzed by the enrichment compared to input method.

HUDEP-2 cell culture and differentiation, Cas9 RNP Nucleofection, and in vitro transcription of gRNA were performed as generally described above.

Results of this experiment are shown in Fig. 6.

This example shows that the inserted sequences are bound by human KLF1 and TFIIB proteins.

ChIP-qPCR primer sequences are shown below (forward primer (top) and reverse primer (bottom) shown for each gene, respectively).
HBB:
   SEQ ID NO: 82: CCAACTCCTAAGCCAGTGCCAGAA
   SEQ ID NO: 83: CAAATGTAAGCAATAGATGGCTCTGCC
HBD:
   SEQ ID NO: 84: GGGCAAGTTAAGGGAATAGTGGAATGAAGG
   SEQ ID NO: 85: GTCAGAAGAAAGTGTAAGCAACAGTCGAC
SP1:
   SEQ ID NO: 86: ACCTCTCCGCCCACTAGGA
   SEQ ID NO: 87: CAACGGCCAACCAGAATCC
AREG:
   SEQ ID NO: 88: TCCACTTCCTCTCAGCGAAT
   SEQ ID NO: 89: GGTGTGCGAACGTCTGTAGG
VEGFA:
   SEQ ID NO: 90: GGTTTGTATCCTGCCCTTCC
   SEQ ID NO: 91: ACTGGGTCTTGCTGTTTTCC

### Example 6. HBD genome editing of CD34+ human hematopoietic stem and progenitor cells (HSPCs).

Mobilized peripheral blood hematopoietic stem and progenitor cells (mPB-HSPCs) cell culture and differentiation was performed as described below.

All cell culture was performed at 37°C in a humidified atmosphere containing 5% CO₂.

For editing for human CD34+ cells, CD34+ mobilized peripheral blood HSPCs were thawed and cultured in SFEM containing CC110 supplement (Stemcell Technologies 02697) for 2 days. CD34+ cells were then electroporated with RNP nucleofection and transferred into erythroid expansion media containing SFEM and erythroid expansion supplement (Stemcell Technologies 02692). After 2 days, the cells were single-cell plated to obtain clonal colonies. After 7 days of expansion, the resulting early erythroblasts were transferred to differentiation media containing SFEM with 50ng/ml erythropoietin, 3% normal human serum, and 1 µM) mifepristone. The resulting late erythroblasts were harvested for analysis after 5 days of differentiation.

Individual colonies were isolated from editing CD34+ human HSPCs with (Cas9 + gRNA+ DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55)). Analysis by qRT-PCR was performed as described above. Colonies were genotyped by NGS as described in Example 2.

Results are shown in Fig. 7. This example shows efficient expression of delta globin from the human *HBD* gene after genome editing using the inventive CRISPR/Cas composition in CD34+ hematopoietic stem cells and their successful differentiation into erythroblasts.

### Example 7. HBD gene editing efficiencies of different gRNAs

HUDEP-2 cells were edited with 2 different gRNAs and editing outcomes were compared.

The following gRNAs were used together with SpCas9 (Cas9 RNP Nucleofection) as described above:
1. gRNA 1 (SEQ ID NO: 46; comprises DNA targeting segment having SEQ ID NO: 23)
2. gRNA 2 (SEQ ID NO: 47; comprises DNA targeting segment having SEQ ID NO: 26)

Each of these gRNA/Cas9 RNPs were tested both in the presence and absence of a DNA donor template containing KLF1 and TFIIB binding sites and the b-DRF motif (KLF1 binding site having SEQ ID NO: 52, b-DRF motif having SEQ ID NO: 54 and TFIIB binding site having SEQ ID NO: 53; DNA donor template having SEQ ID NO: 55).

Cells were harvested for NGS sequencing as described above (cf. example 2) and Synthego ICE analysis was used to assign editing outcomes of percent unmodified, NHEJ, and HDR.

This example shows that both gRNAs are suitable in the context of the present invention. Best results were obtained with gRNA 2 comprising the DNA targeting segment having SEQ ID NO: 26. Results of this example are shown in Fig. 8.

## Claims

1. A CRISPR/Cas composition or a kit for editing a human HBD gene comprising:
(a) a first component selected from
(a-1) a guide RNA (gRNA) comprising a DNA targeting segment directed to the promoter region of the human HBD gene, preferably comprising a nucleotide sequence selected from the group consisting of SEQ ID NOs: 1 - 43, wherein the DNA targeting segment has a length of between 20 to 24 nucleotides, and
(a-2) a DNA polynucleotide encoding said gRNA, optionally wherein said DNA polynucleotide encoding said gRNA is comprised in a vector, preferably an AAV vector or a lentiviral vector;
(b) a second component selected from
(b-1) a DNA donor template, and
(b-2) a vector, preferably an AAV vector or a lentiviral vector comprising the DNA donor template,
wherein the DNA donor template comprises
(b-i) a first homology arm sequence having at least 80% sequence identity to a first portion of a chromosomal DNA sequence comprised in the promoter region of the human *HBD* gene,
(b-ii) a second homology arm sequence having at least 80% sequence identity to a second portion of a chromosomal DNA sequence comprised in the promoter region of the human HBD gene,
(b-iii) a human KLF1 binding site, preferably having SEQ ID NO: 52,
(b-iv) a human TFIIB binding site, preferably having SEQ ID NO: 53, and
(b-v) a human b-DRF motif, preferably having SEQ ID NO: 54,
wherein
the first homology arm sequence and the second homology arm sequence independently of each other have a length of between 20 to 200 nucleotides, and
the human KLF1 binding site, the human TFIIB binding site, and the human b-DRF motif are located 3' of the first homology arm sequence and 5' of the second homology arm sequence; and
(c) a third component selected from
(c-1) a Cas9 polypeptide or a variant thereof, and
(c-2) a nucleic acid encoding a Cas9 polypeptide or a variant thereof, optionally comprised in a vector, preferably an AAV vector or a lentiviral vector.

2. The CRISPR/Cas composition or the kit according to claim 1, wherein
(a) the gRNA or the DNA polynucleotide encoding said gRNA;
(b) the DNA donor template or the vector comprising the DNA donor template sequence, and
(c) the Cas9 polypeptide or the variant thereof, or the nucleic acid encoding said Cas9 polypeptide or the variant thereof
are present in a cell *in vitro* or ex *vivo,* wherein said cell is not a human germ cell.

3. The CRISPR/Cas composition or the kit according to claim 1 or 2, wherein the gRNA is chemically modified, preferably modified at the 2' position of the sugar, particularly modified with modifications selected from the group consisting of 2'-OMe, 2'-MOE and 2'-F modifications, and / or modified at the phosphate group, particularly modified with phosphorothioate and / or phosphorodithioate modifications.

4. The CRISPR/Cas composition or the kit according to any one of claims 1 to 3 comprising:
(a-1) the gRNA comprising a DNA targeting segment directed to the promoter region of a human HBD gene, preferably comprising a nucleotide sequence selected from the group consisting of SEQ ID NOs: 1 - 43, wherein the DNA targeting segment has a length of between 20 to 24 nucleotides, and
(b-1) the DNA donor template, wherein the DNA donor template comprises
(b-i) a first homology arm sequence having at least 80% sequence identity to a first portion of a chromosomal DNA sequence comprised in the promoter region of the human HBD gene,
(b-ii) a second homology arm sequence having at least 80% sequence identity to a second portion of a chromosomal DNA sequence comprised in the promoter region of the human HBD gene,
(b-iii) a human KLF1 binding site, preferably having SEQ ID NO: 52,
(b-iv) a human TFIIB binding site, preferably having SEQ ID NO: 53, and
(b-v) a human b-DRF motif, preferably having SEQ ID NO: 54,
wherein
the first homology arm sequence and the second homology arm sequence independently of each other have a length of between 20 to 200 nucleotides, and
the human KLF1 binding site, the human TFIIB binding site, and the human b-DRF motif are located 3' of the first homology arm sequence and 5' of the second homology arm sequence; and
(c-1) the Cas9 polypeptide or the variant thereof.

5. A gRNA comprising a DNA targeting segment directed to the promoter region of the human HBD gene, preferably comprising a nucleotide sequence selected from the group consisting of SEQ ID NOs: 1 - 43, wherein the DNA targeting segment has a length of between 20 to 24 nucleotides; or
a DNA polynucleotide encoding said gRNA, optionally wherein said DNA polynucleotide encoding said gRNA is comprised in a vector, preferably an AAV vector or a lentiviral vector.

6. The gRNA according to claim 5, wherein the gRNA is chemically modified, preferably modified at the 2' position of the sugar, particularly modified with modifications selected from the group consisting of 2'-OMe, 2'-MOE and 2'-F modifications, and / or modified at the phosphate group, particularly modified with phosphorothioate and / or phosphorodithioate modifications.

7. A DNA donor template, or a vector, preferably an AAV vector or a lentiviral vector, comprising a DNA donor template, wherein the DNA donor template comprises
(i) a first homology arm sequence having at least 80% sequence identity to a first portion of a chromosomal DNA sequence comprised in the promoter region of a human HBD gene,
(ii) a second homology arm sequence having at least 80% sequence identity to a second portion of a chromosomal DNA sequence comprised in the promoter region of a human HBD gene,
(iii) a human KLF1 binding site, preferably having SEQ ID NO: 52,
(iv) a human TFIIB binding site, preferably having SEQ ID NO: 53, and
(v) a human b-DRF motif, preferably having SEQ ID NO: 54,
wherein the first and the second homology arm sequence have independently of each other a length of between 20 to 200 nucleotides, and
wherein the human KLF1 binding site, the human TFIIB binding site, and the human b-DRF motif are located 3' of the first homology arm sequence and 5' of the second homology arm sequence.

8. A nucleic acid comprising:
(A) a first nucleotide sequence encoding a gRNA comprising a DNA targeting segment directed to the promoter region of the human HBD gene, preferably comprising a nucleotide sequence selected from the group consisting of SEQ ID NOs: 1 - 43, wherein the DNA targeting segment has a length of between 20 to 24 nucleotides,
wherein the first nucleotide sequence encoding said gRNA is operably linked to a promoter; and/or
(B) a second nucleotide sequence comprising a DNA donor template, wherein the DNA donor template comprises
(B-i) a first homology arm sequence having at least 80% sequence identity to a first portion of a chromosomal DNA sequence comprised in the promoter region of a human HBD gene,
(B-ii) a second homology arm sequence having at least 80% sequence identity to a second portion of a chromosomal DNA sequence comprised in the promoter region of a human HBD gene,
(B-iii) a human KLF1 binding site, preferably having SEQ ID NO: 52,
(B-iv) a human TFIIB binding site, preferably having SEQ ID NO: 53, and
(B-v) a human b-DRF motif, preferably having SEQ ID NO: 54,
wherein
the first homology arm sequence and the second homology arm sequence independently of each other have a length of between 20 to 200 nucleotides, and
the human KLF1 binding site, the human TFIIB binding site, and the human b-DRF motif are located 3' of the first homology arm sequence and 5' of the second homology arm sequence.

9. The nucleic acid according to claim 8, further comprising (C) a third nucleotide sequence encoding a Cas9 polypeptide or a variant thereof, wherein the third nucleotide sequence is operably linked to a promoter.

10. The nucleic acid according to claim 8 or 9, wherein said nucleic acid is a recombinant expression vector, preferably a viral vector, more preferably an AAV vector or a lentiviral vector.

11. The CRISPR/Cas composition or the kit for editing a human HBD gene according to any one of claims 1 to 4 or
a gRNA or a DNA polynucleotide encoding said gRNA according to claim 5 or 6, or
a DNA donor template or a vector comprising a DNA donor template according to claim 7, or
a nucleic acid according to any one of claims 8 to 10,
for use in medicine.

12. The CRISPR/Cas composition or the kit for editing a human HBD gene according to any one of claims 1 to 4; or
a gRNA or a DNA polynucleotide encoding said gRNA according to claim 5 or 6, or
a DNA donor template or a vector comprising a DNA donor template according to claim 7, or
a nucleic acid according to any one of claims 8 to 10,
for use in the treatment of a haemoglobinopathy.

13. The CRISPR/Cas composition for use or the kit for editing a human HBD gene for use according to claim 12; or
the gRNA or the DNA polynucleotide encoding said gRNA for use according to claim 12; or
the DNA donor template or the vector comprising a DNA donor template for use according to claim 12; or
the nucleic acid for use according to claim 12,
wherein the haemoglobinopathy is selected from sickle cell disease and beta thalassemia.

14. A method of editing a human HBD gene in a cell *in vitro* or ex *vivo,* the method comprising:
(a) Introducing into the cell, simultaneously or successively,
(a-i) the gRNA as defined in claim 5 or 6 or the DNA polynucleotide encoding said gRNA as defined in claim 1;
(a-ii) the Cas9 polypeptide or the variant thereof as defined in claim 1 or the polynucleotide encoding said Cas9 polypeptide or the variant thereof as defined in claim 1; and
(a-iii) the DNA donor template as defined in claim 6 or the vector comprising the DNA donor template as defined in claim 7; and
(b) culturing the cell.

15. The method according to claim 14, wherein the cell is a CD34+ hematopoietic stem cell.
